# EUROPEAN PATENT APPLICATION

(11) **EP 4 328 314 A2**
(43) Date of publication of application: **28.02.2024**
(21) Application number: 23203088.2
(22) Date of filing: 21.08.2018
(51) Int. Cl.: C12N 15/88

(54) **LIPID NANOPARTICLE METHODS AND COMPOSITIONS FOR PRODUCING ENGINEERED ERYTHROID CELLS**

(30) Priority: 22.08.2017 US 201762548797 P
(62) Divisional of application: 18779837.6
(71) Applicant: Tessera Therapeutics, Inc., Somerville, MA 02143 (US)
(72) Inventor: HARANDI, Omid, Cambridge 02139 (US); KAHVEJIAN, Avak, Cambridge 02139 (US)
(74) Representative: Maiwald GmbH

(57) **Abstract**

Carriers such as lipid nanoparticles are useful for delivering payloads (such as RNA, DNA, proteins, or small molecules) to cells such as erythroid cells. This disclosure provides improved carriers that comprise a targeting agent that binds a cell surface receptor. In some embodiments, the cell surface receptor is one that undergoes a high rate of endocytosis.

## Description

### RELATED APPLICATIONS

This application claims priority to U.S. Serial No. 62/548,797 filed Aug 22, 2017, the contents of which are incorporated-herein by reference in their entirety.

### SEQUENCE LISTING

The instant application contains a Sequence Listing, which has been filed electronically in ASCII format and is hereby incorporated by reference in its entirety. Said ASCII copy, created on August 21, 2018, is named R2081-7030WO_SL.txt and is 14,439 bytes in size.

### BACKGROUND

Erythroid cells such as red blood cells can be engineered to express a wide variety of exogenous therapeutic agents. This engineering can involve introducing a transgene into erythroid cell precursors, and then inducing the precursors to differentiate and express the transgene. However, not every stage of erythroid differentiation is equally amenable to the introduction of nucleic acids. There exists a need for improved method of introducing nucleic acids into erythroid cells.

### SUMMARY OF THE INVENTION

When delivering mRNA or protein to differentiating erythroid cells, it can (in some embodiments) be desirable to deliver the mRNA or protein late in differentiation, so that subsequent rounds of cell division do not drastically reduce the amount of mRNA or protein present in the cell. However, some delivery methods have lower efficiency late in erythroid cell differentiation. Without wishing to be bound by theory, a decline in the levels of certain cell surface receptors, like LDL-R (see Example 5 herein) during differentiation can lead to lower transfection efficiency of methods that rely on such receptors. The compositions and methods herein reflect (at least in part) the appreciation that other cell surface receptors remain present and actively internalized even late into erythroid cell differentiation. Transferrin receptor is one such receptor (see Example 6 herein). As described herein, various payloads can be delivered to the cell via a receptor of this type. As shown in Example 8 herein, a carrier comprising a targeting agent that binds the transferrin receptor successfully delivers a nucleic acid to the erythroid cell, allowing the erythroid cell to translate the nucleic acid at high levels.

Accordingly, the present disclosure describes carriers such as lipid nanoparticles that are useful for delivering payloads (such as RNA, DNA, proteins, or small molecules) to cells such as erythroid cells. For instance, the disclosure provides improved carriers that comprise a targeting agent that binds a cell surface receptor. In some embodiments, the cell surface receptor is one that undergoes a high rate of endocytosis. Without wishing to be bound by theory, once the carrier binds to the cell surface receptor, it can be rapidly internalized through an endocytic pathway, efficiently introducing the payload into the cell.

The present disclosure provides, in some aspects, an in *vitro* method of introducing a payload into an erythroid cell, comprising:
contacting an erythroid cell with a carrier, e.g., a nanoparticle, which carrier comprises:
   a) a payload, and
   b) a targeting agent that binds a cell surface receptor of an erythroid cell,
under conditions that allow uptake of the payload into the erythroid cell,
thereby introducing the payload into the erythroid cell.

The present disclosure provides, in some aspects, a method, e.g., an *in vitro* method, of introducing a nucleic acid into an erythroid cell, comprising:
contacting an erythroid cell with a carrier, e.g., a nanoparticle, which carrier comprises:
   a) the nucleic acid, and
   b) a targeting agent that binds a cell surface receptor of an erythroid cell,
under conditions that allow uptake of the nucleic acid into the erythroid cell,
thereby introducing the nucleic acid into the erythroid cell.

The present disclosure provides, in some aspects, an *in vitro* method of introducing a nucleic acid (e.g., mRNA) into an erythroid cell, comprising:
contacting an erythroid cell with a nanoparticle which comprises:
   a) the nucleic acid, and
   b) a polypeptide targeting agent that binds a cell surface receptor (e.g., CD71) of an erythroid cell,
under conditions that allow uptake of the nucleic acid into the erythroid cell,
thereby introducing the nucleic acid into the erythroid cell.
The present disclosure provides, in some aspects, an in *vitro* method of introducing an mRNA into an erythroid cell, comprising:
contacting an erythroid cell with an LNP, which LNP comprises:
   a) the mRNA, and
   b) a CD71binding targeting agent that comprises an amino acid sequence of HAIYPRH (SEQ ID NO: 9) or an amino acid sequence having at least 70%, 85%, 80%, 85%, 90%, 95%, 96%, 97%, 98%, or 99% identity thereto,
under conditions that allow uptake of the mRNA into the erythroid cell,
thereby introducing the mRNA into the erythroid cell.

The present disclosure provides, in some aspects, a method of making a functionalized cell, comprising:
contacting a cell (e.g., an erythroid cell) with a carrier, e.g., a nanoparticle, which carrier comprises:
   a) a payload (e.g. a nucleic acid or a polypeptide), and
   b) a targeting agent that binds a cell surface receptor of the cell,
under conditions that allow uptake of the payload into the cell,
thereby making the functionalized cell.

The present disclosure provides, in some aspects, a reaction mixture comprising:
a) a carrier, e.g., a nanoparticle, comprising a payload (e.g., a nucleic acid) and a targeting agent that binds a cell surface receptor of an erythroid cell,
b) an erythroid cell comprising the cell surface receptor, said cell surface receptor capable of mediating internalization of the carrier.

In some aspects, the disclosure provides a reaction mixture comprising:
a) a nanoparticle comprising a nucleic acid and a polypeptide targeting agent that binds a cell surface receptor, (e.g., CD71) of an erythroid cell,
b) an erythroid cell comprising the cell surface receptor, said cell surface receptor capable of mediating internalization of the carrier.

In some aspects, the disclosure provides a reaction mixture comprising:
a) a carrier, e.g., nanoparticle, comprising a payload (e.g., a nucleic acid) and a targeting agent that binds a cell surface receptor-of a cell (e.g., an erythroid cell).
b) a cell (e.g., an erythroid cell) comprising the cell surface receptor, said cell surface receptor capable of mediating internalization of the carrier,
wherein the reaction mixture is situated in a bioreactor, e.g., a bioreactor described herein.

In some aspects, the disclosure provides a carrier, e.g., a nanoparticle, comprising:
a) a nucleic acid, e.g., mRNA, and
b) a targeting agent that binds a cell surface receptor of an erythroid cell, wherein one or more of:
   i) the targeting agent is a CD71-binding fragment of transferrin or analog of transferrin, e.g., is less than 600, 500, 400, 300, 200, 100, 50, 40, 30, 20, or 10 amino acids in length;
   ii) the targeting agent is not covalently linked to PEG;
   iii) the nucleic acid is RNA, e.g., mRNA;
   iv) the nucleic acid does not encode a fluorescent protein, e.g., GFP, e.g., EGFP; or
   v) the carrier does not comprise one or both of stearic acid and soya lethicin.

In some aspects, the disclosure provides a carrier, e.g., a nanoparticle, comprising:
a) mRNA, and
b) a targeting agent that binds a cell surface receptor of an erythroid cell.

In some aspects, the disclosure provides a carrier, e.g., a nanoparticle, comprising:
a) a payload, and
b) a targeting agent described herein, e.g., a targeting agent that binds a cell surface receptor of an erythroid cell, wherein the targeting agent is a CD71-binding fragment of transferrin or analog of transferrin, e.g., is less than 600, 500, 400, 300, 200, 100, 50, 40, 30, 20, or 10 amino acids in length.

In some aspects, the disclosure provides an erythroid cell comprising a carrier (e.g., nanoparticle) described herein, e.g., wherein the carrier is disposed at the cell surface, or internal to the cell, e.g., in an endocytic vesicle, in an endosome, or in the cytoplasm.

In some aspects, the present disclosure also provides an erythroid cell comprising a nanoparticle described herein.

In some aspects, the present disclosure also provides an erythroid cell made by a process described herein.

In some aspects, the disclosure provides an erythroid cell that comprises a payload, made by a process comprising:
contacting an erythroid cell with a carrier, e.g., a nanoparticle, which carrier comprises:
   a) the payload. and
   b) a targeting agent that binds a cell surface receptor of an erythroid cell,
under conditions that allow uptake of the payload into the erythroid cell.

In some aspects, the disclosure provides a preparation comprising at least 10⁵, 10⁶, 10⁷, 10⁸, 10⁹, 10¹⁰, 10¹¹, 10¹², or 10¹³ erythroid cells described herein.

Any of the aspects herein, e.g., aspects above, can be combined with one or more of the embodiments, described herein, e.g., the embodiments below.

In some embodiments, the targeting agent is a polypeptide.

In some embodiments, the cell surface receptor is a protein that undergoes endocytosis at a rate of about 200-800, 300-700, 400-600, or 500 molecules/cell/second, e.g., as measured by a radiolabeling assay of Iaeopetta BJ, Morgan EH. The kinetics of transferrin endocytosis and iron uptake from transferrin in rabbit reticulocytes. J Biol Chem. 1983 Aug 10;258(15):9108-15).

In some embodiments, the cell surface receptor is a protein that comprises an internalization signal, e.g., NPXY, FXNPXY (SEQ ID NO: 5), YXXL, [D/E]XXXL[L/I], YXXQ, GDXY, YXXphi (wherein phi is an amino acid residue with a hydrophobic side chain, e.g., V, I, L, F, W, Y or M), or a dileucine-dependent signal-sequence motif such as DKphiTLL (SEQ ID NO: 6), LLHV (SEQ ID NO: 7), HLLPM (SEQ ID NO: 8), and DXXLL. In embodiments; the internalization signal is disposed in a cytoplasmic domain of the cell surface receptor. In some embodiments, the internalization motif is a motif that binds a clathrin-associated sorting proteins such as adaptor protein-2 (AP-2), e.g., wherein the internalization motif is [D/E]XXXL[L/I] or YXXQ. In some embodiments, the internalization motif is a motif that binds clathrin.

In some embodiments, the cell surface receptor is a protein in the clathrin-mediated endocytic pathway, caveolae-mediated endocytic pathway, or macropinocytosis endocytic pathway.

In embodiments, the cell surface receptor is a type I red blood cell transmembrane protein (e.g., EPO Receptor or glycophorin A), a type II red blood cell transmembrane protein (e.g., CD71 or Kell), or a type III red blood cell transmembrane protein, such as glucose transporter 1 (GLUT1).

In some embodiments, the cell surface receptor is CD71. In some embodiments, the targeting agent comprises transferrin or a CD71-binding fragment or analog thereof. In some embodiments, the targeting agent is a CD71-binding fragment of transferrin or analog of transferrin.

In some embodiments, the targeting agent binds iron. In some embodiments, the targeting agent does not bind iron.

In some embodiments, the targeting agent is less than 600, 500, 400, 300, 200, 100, 50, 40, 30, 20, or 10 amino acids in length.

In some embodiments, the targeting agent comprises an amino acid sequence of HAIYPRH (SEQ ID NO: 9) or THRPPMWSPVWP (SEQ ID NO: 10), or a sequence with no more than 1, 2, or 3 alterations relative thereto, e.g., wherein an alteration comprises an insertion, deletion, or substitution or any combination thereof. In some embodiments, the targeting agent comprises an anti-CD71 antibody molecule. In some embodiments, the targeting agent is linked to the carrier (e.g., to a lipid or PEG of the carrier) by its N-terminus, its C-terminus, or by an internal residue.

In some embodiments, the cell surface receptor is Erythropoietin receptor (EPO-R). In some embodiments, the targeting agent comprises an EPO molecule, e.g., an EPO molecule described herein.

In some embodiments, the carrier, e.g., nanoparticle, further comprises a second targeting agent, e.g., a targeting agent described herein. In some embodiments, the second targeting agent binds the first cell surface receptor or a second cell surface receptor, e.g., wherein the cell surface receptor is a cell surface receptor described herein. In some embodiments, the cell surface receptor is an endogenous polypeptide. In some embodiments, the cell surface receptor is an exogenous polypeptide.

In some embodiments, the targeting agent is not covalently linked to PEG, e.g., to PEG-PE. In some embodiments, the targeting agent is covalently linked to PEG, e.g., to PEG-PE.

In some embodiments, the payload comprises a nucleic acid, e.g., DNA or RNA, e.g., an mRNA. In some embodiments, the RNA comprises one or both of a 5' cap and a 3' poly-A tail. In some embodiments, the DNA comprises a lentiviral DNA sequence.

In some embodiments, the payload comprises a polypeptide. In some embodiments, the polypeptide is a soluble protein, a transmembrane protein, or a lipid-anchored protein.

In some embodiments, the payload comprises a small molecule. In some embodiments, the small molecule is a chemotherapeutic agent, e.g., a chemotherapeutic agent described herein.

In some embodiments, the payload comprises or encodes an enzyme, antibody molecule, cytokine, receptor, transporter, or functional fragment of any of the foregoing.

In some embodiments, the payload comprises a nucleic acid (e.g., an mRNA) that encodes a polypeptide comprising an enzyme or an enzymatically active fragment or variant thereof. In some embodiments, the enzyme comprises phenylalanine lyase (PAL), uricase, cystathionine beta synthase (CBS), oxalate oxidase, aminolevulinate dehydrogenase, or asparaginase. In some embodiments, the polypeptide encodes a transmembrane domain, e.g., a fusion protein comprising a transmembrane domain and the enzyme or fragment or variant thereof, e.g., for expression on the cell surface. In some embodiments, the polypeptide is one expressed in the cell interior, e.g., in the cell interior and not associated with the cell membrane.

In some embodiments, the payload comprises a nucleic acid (e.g., an mRNA) that encodes a polypeptide comprising an antigen. The antigen may comprise, e.g., an autoimmune disease antigen or a tumor antigen. In some embodiments, the antigen comprises a full-length protein or a fragment thereof. In some embodiments, the antigen has a wild-type sequence, and in other embodiments, the antigen has one or more mutations relative to the corresponding wild-type sequence. In some embodiments the antigen comprises a MOO antigen (e.g., MOG 35-55 peptide), a preproinsulin antigen, a proinsulin antigen, an insulin antigen (e.g., amino acids 9-23 of insulin B-chain), a clotting factor antigen, an ADAMTS13 antigen, a cadherin antigen, an acetylcholine receptor antigen, an Aquaporin 4 antigen, or a PLA2R antigen. In some embodiments, the tumor antigen comprises a NY-ESO-1 antigen, a MAGE-A antigen, or a hTERT antigen. In some embodiments, the polypeptide encodes a transmembrane domain, e.g., a fusion protein comprising a transmembrane domain and the antigen, e.g., for expression on the cell surface. In some embodiments, the polypeptide is one expressed in the cell interior, e.g., in the cell interior and not associated with the cell membrane.

In some embodiments, the payload comprises a nucleic acid (e.g., an mRNA) that encodes a polypeptide comprising a cell surface receptor or active fragment or variant thereof. In some embodiments, the cell surface receptor is chosen from a cytokine receptor (e.g., IL15Ra), anMHC Iprotein (e.g., bound to an antigen), or an MHC II protein (e.g., bound to an antigen), or an active fragment or variant thereof. In some embodiments, the polypeptide encodes a transmembrane domain (e.g., a transmembrane domain that is endogenous or exogenous to the receptor), e.g., for expression on the cell surface.

In some embodiments, the payload comprises a nucleic acid (e.g., an mRNA) that encodes a polypeptide comprising a ligand. In some embodiments, the ligand comprises a costimulatory protein or an active fragment or variant thereof. In some embodiments, the ligand is chosen from 4-1BBL, OX40-L, ICOS-L, or GITR-L, or an active fragment or variant thereof. In some embodiments, the polypeptide encodes a transmembrane domain, e.g., a fusion protein comprising a transmembrane domain and the ligand, e.g., for expression on the cell surface.

In some embodiments, the payload comprises a nucleic acid (e.g., an mRNA) that encodes a polypeptide comprising a cytokine or active fragment or variant thereof. In some embodiments, the cytokine is a part of a fusion protein, e.g., a fusion protein comprising the cytokine and a cytokine receptor, e.g., IL-15TP (a fusion of IL-15 and IL-15 receptor alpha). In some embodiments, the cytokine comprises IL-15, IL-12, IL-18, or IL-21. In some embodiments, the polypeptide encodes a transmembrane domain, e.g., a fusion protein comprising a transmembrane domain and the cytokine, e.g., for expression on the cell surface.

In some embodiments, the payload comprises a nucleic acid (e.g., an mRNA) that encodes a polypeptide comprising a modulator of a checkpoint inhibitor protein or active fragment or variant thereof. In some embodiments, the modulator of a checkpoint inhibitor protein comprises an anti-PDl antibody molecule, and anti-PDLl antibody molecule, or an anti-CTLA4 antibody molecule. In some embodiments, the polypeptide encodes a transmembrane domain, e.g., a fusion protein comprising a transmembrane domain and the antibody molecule, e.g., for expression on the cell surface.

In some embodiments, the payload comprises a nucleic acid (e.g., an mRNA) that encodes a polypeptide comprising an antibody molecule, e.g,, an scFv. In some embodiments, the antibody molecule binds to CD20, PD1, EpCAM, CD33, CD38, orCD123. In some embodiments, the antibody molecule is a targeting agent. In some embodiments, the polypeptide encodes a transmembrane domain, e.g., a fusion protein comprising a transmembrane domain and the antibody molecule, e.g., for expression on the cell surface.

In some embodiments, the carrier further comprises a second payload. In some embodiments, the payload comprises a nucleic acid, e.g., RNA, and the second payload comprises a second nucleic acid, e.g., a second RNA. In,some embodiments, the payload comprises a polypeptide, and the second payload comprises a second polypeptide. In some embodiments, the payload comprises a small molecule, and the second payload comprises a second small molecule. In some embodiments, the payload comprises a nucleic acid, e.g., RNA, and the second payload comprises a polypeptide. In some embodiments, the payload comprises a nucleic acid, e.g., RNA, and the second payload, comprises a small molecule. In some embodiments, the payload comprises a polypeptide, and the second payload comprises a small molecule. In some embodiments, the carrier further comprises a third payload, e.g., a payload described herein. In some embodiments, the carrier comprises at least 4, 5, 10, 20, 50, or 100 different payloads, e.g., payloads described herein.

In some embodiments, the payload comprises an enzyme (e.g., asparaginase or PAL) and the second payload comprises a transporter for a substrate or metabolite of the enzyme. In some embodiments, the transporter is an amino acid transporter, e.g., an asparagine transporter (e.g., an SN2 or SAT2 polypeptide), a phenylalanine transporter polypeptide (e.g., a TATI polypeptide), or a L-homocysteine or L-serine transporter (e.g., ASCT1).

In some embodiments, the nanoparticle is a lipid nanoparticle (LNP), solid lipid nanoparticle (SLN), nanostructured lipid carrier (NLC), lipoplex nanoparticle, polyplex nanoparticle, polymer nanoparticle, or hybrid lipid/polymer nanoparticle.

In some embodiments, the carrier, e.g., nanoparticle, comprises an ionizable lipid or a cationic lipid, e.g., as described herein, In some embodiments, the carrier, e.g., nanoparticle, comprises stearic acid or 3C. In some embodiments, the carrier, e.g., nanoparticle, comprises cholesterol or soya lethicin.

In some embodiments, the nucleic acid is disposed in the core of the nanoparticle. In some embodiments, the nucleic acid is disposed at the surface of the nanoparticle. In some embodiments, thenucleic acid is non-covalently bound to the carrier, e.g., nanoparticle.

In some embodiments, the targeting agent is bound (e.g., covalently or non-covalently bound) to the surface of the carrier, e.g., nanoparticle.

In some embodiments, the nanoparticle further comprises a polyether, e.g., PEG (polyethylene glycol), e.g., lipid-anchored PEG, e.g., PEG-PE (PEG-phosphatidylethanolamine). In embodiments, the PEG is PEG-2000. In embodiments, the carrier, e.g., LNP, comprises a lipid-PEG molecule, e.g., a DSPE-PEG molecule. In some embodiments, the DSPE-PEG molecule comprises DSPE-PEG 5000, e.g., DSPE-PEG 5000-Azide, e.g., having the structure:

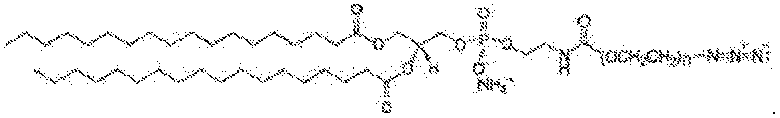

In embodiments, the DSPE-PEG molecule comprises PEG 2000, e.g., PEG 2000-Azide, e.g., 16:0 PEG 2000-Azide.

In embodiments, the carrier, e.g., nanoparticle comprises a polar lipid, e.g., a cationic lipid, e.g., a cationic lipid described herein. In-embodiments, the carrier, e.g., nanoparticle, comprises a lipid bilayer, e.g., a lipid bilayer having a lumen. In embodiments, the carrier comprises a lumen and the payload, e.g., nucleic acid, is disposed in the lumen.

In embodiments, the targeting agent is attached to a surface moiety comprised by the carrier, e.g., nanoparticle.

In embodiments, the nucleic acid is disposed within the carrier and the targeting agent is disposed on the surface of the carrier.

In embodiments, the carrier is capable of transfecting at least 10%, 20%, 30%, 40%, 50%, 60%, 70%, 80%, or 90% of cells in the reaction mixture, e.g.., by an assay of Example 1.

In embodiments, the carrier does not comprise one or more of: a chemotherapeutic agent, e.g., doxorubicin; folate; a detergent, e.g., Tween-80; DDAB; precirol, e.g., precirol ATO-5; olive oil; lipoid, e.g., lipoid S-100; or 6-lauroxyhexyl omithinate (LHON). In embodiments, the carrier does not include a chemotherapeutic agent, e.g., a mitotic inhibitor or spindle poison, e.g., a taxane, e.g., docetaxel or paclitaxel; an aromatase inhibitor, e.g., 7a-APT ADD; a DNA replication inhibitor, e.g., a topoisomerase inhibitor, e.g., an anthracycline, e.g., doxorubicin; or a pro-apoptotic agent, e.g., artemisinin. In embodiments, the carrier does not include a nucleic acid encoding a fluorescent protein e.g., luciferase or GFP, e.g., EGFP. In embodiments, the carrier does not comprise one or both of stearic acid and soya lethicin.

In embodiments, the reaction mixture further comprises a solvent and the carrier is insoluble in the solvent, e.g., forms an emulsion in the solvent.

In embodiments, at least 50%, 60%, 70%, 80%, 90%, 95%, 96%, 97%, 98%, or 99% of the cells in the reaction mixture are erythroid cells, In embodiments, at least 50%, 60%, 70%, 80%, 90%, 95%, 96%, 97%, 98%, or 99% of the cells in the reaction mixture are myeloid, lineage cells. In embodiments, less than 50%, 40%, 50%, 20%, 10%, 5%, 4%, 3%, 2%, or 1% the cells in the reaction mixture are other than erythroid cells, e.g., are transformed cells, cancer cells, lymphoid lineage cells, or white blood cells (e.g., T cells), or a combination thereof. In embodiments, the reaction mixture is substantially free of one or more of transformed cells, cancer cells, lymphoid lineage cells, or white blood cells (e.g., T cells).

In embodiments, the reaction mixture comprises at least 10⁵, 10⁶, 10⁷, 10⁸, 10⁹, 10¹⁰, 10¹¹, 10¹², or 10¹³ erythroid cells. In embodiments, the reaction mixture has a volume of at least 1 ml, 2 ml, 5 ml, 10 ml, 20 ml, 50 ml, 100 ml, 200 ml, 500 ml, 1L, 2 L, 5 L, 10 L, 20 L, 50 L, 100 L, 200 L, or 500 L.

In embodiments, the reaction mixture is in a bioreactor. In embodiments, the reaction mixture is under GMP conditions. In embodiments, the reaction mixture lacks detectable levels of one or more pathogens, e.g., bacteria, viruses, or fungi.

In embodiments, the erythroid cell is in differentiation phase or maturation phase. In some embodiments, the erythroid cell is in day M1-M6, M1-M5, M1-M4, or M2-M4 of maturation phase. In some embodiments, the erythroid cell is in day M1, M2, M3, M4, M5, or M6 of maturation phase. In some embodiments, the carrier, e.g., LNP, is contacted to the erythroid cell in differentiation phase or maturation phase. In some embodiments, the carrier, e.g., LNP, is contacted to the erythroid cell at day M1-M6, M1-M5, M1-M4, or M2-M4 of maturation phase. In some embodiments, the carrier, e.g., LNP, is contacted to the erythroid cell at day M1, M2, M3, M4, M5, or M6 of maturation phase.

In embodiments, the erythroid cell is an erythroid progenitor cell. In embodiments, the erythroid cell is an erythroid precursor cell. In embodiments, the erythroid cell is a pro-erytproblast. In embodiments, the erythroid cell is a basophilic erythroblast, normoblast (e.g., polychromatic or orthochromatic normoblast) or reticulocyte.

In embodiments, the method comprises providing a population of cells, or the reaction mixture comprises a population of cells, and one or more cells of the population are erythroid cells, e.g., erythroid cells described herein.

In embodiments, at the time the cells in the population are contacted with the carrier, one or more of:
at least 50%, 60%, 70%, 80%, 85%, 90%, 95%, or 99% of the cells in the population are erythroid progenitors, e.g., pro-erythroblasts;
30-90%, 40-90%, 50-90%, 60-90%, or 70-90% of the cells in the population are erythroid progenitors, e.g., pro-erythroblasts;
at least 50%, 60%, 70%, 80%, 85%, 90%, 95%, or 99% of the cells in the population exhibit the morphology of an erythroid progenitor, e.g., pro-erythroblast;
30-90%, 40-90%, 50-90%, 60-90%, or 70-90% of the cells in the population exhibit the morphology of an erythroid progenitor, e.g., pro-erythroblast;
less than 20%, 15%, 10%, 5%, 4%, 3%, 2%, or 1% of the cells in the population are enucleated,
the population of cells will not reach 20%, 30%, 40%, 50%, 60%, or 70% enucleation for at least 48 hours after the cells are contacted with the carrier,
the population of cells will first reach 20%, 30%, 40%, 50%, 60%, or 70% enucleation more than 48 hours after the cells are contacted with the carrier,
0-90%, 82-96%, or about 84% of the cells in the population are CPA-positive (e.g., as measured by a flow cytometry assay, e.g., a flow cytometry assay of Example 4);
40-70%, 45-65%, 50-60%, or about 55% of the cells in the population are band3-positive (e.g., as measured by a flow cytometry assay, e.g., a flow cytometry assay of Example 4);
96-100%, 97-99%, or about 98% of the cells in the population are alpha4 integrin-positive (e.g., as measured by a flow cytometry assay, e.g., a flow cytometry assay of Example 4); or
40-70%, 45-65%, 50-60%, or about 50% or 55% of the cells in the population are alpha4 integrin-positive and band3-positive.

In embodiments, a cell made by a method described herein comprises one or cationic lipids described herein.

In embodiments, a population of cells made by a method described herein comprises less than 50%, 40%, 30%, 20%, 10%, or 5% non-viable cells 24 hours after the contacting step, e.g., wherein viability is measured by counting the total number of cells and contacting the cells with a dye such as Propidium iodide, Draq5, or 7AAD.

In embodiments, a population of cells made by a method described herein comprises less than 20% non-viable or dead cells 1 min, 2 min, 5 min, 10 min, 30 min, 1 hour, 2 hours, 3, hours, 4 hours, 6 hours, 12 hours, or 24 hours after the contacting step.

In embodiments, the method comprises treating the erythroid cells with, or the reaction mixture comprises, one or more exogenous agents that increase the level of a cell surface receptor, e.g., wherein the agent comprises EPO, SCF, IL3. dexamethasone, serum (e.g., human serum), or plasma (e.g., human plasma).

In embodiments, the method does not comprise contacting the erythroid cells with an agent that competes with the targeting agent for binding to the cell surface receptor (a competing agent). In embodiments, the method comprises withdrawing media containing the competing agent and adding media that does not comprise the competing agent or adding media that comprises a lower level of the competing agent compared to the withdrawn media. In embodiments, the cell surface receptor is CD71 and the competing agent is transferrin, e.g., iron-bound transferrin. In embodiments, the cell surface receptor is EPO Receptor and the competing agent is EPO. In some embodiments, the absence of (or lower level of) the competing agent results in increased binding of the targeting agent to the cell surface receptor compared to an otherwise similar reaction mixture when the competing agent is present (or present at a higher level). In some embodiments, the absence of (or lower level of) the competing agent results in increased levels of the cell surface receptor compared to an otherwise similar reaction mixture when the competing agent is present (or present at a higher level). In some embodiments, the lower level of the competing agent is 0-25%, 25-50%, 50-57%, or 75-100% lower than the level of the competing agent in the withdrawn media.

In embodiments, the reaction mixture further comprises a liquid medium capable of supporting erythroid cell metabolism, growth, differentiation, or viability.

In embodiments, the method comprises making the carrier, e.g., nanoparticles, e.g., LNPs. In embodiments, the method comprises making the nanoparticles by combining: a) a cationic lipid, b) a phospholipid, c) lipid-anchored PEG, d) a helper lipid or a structural lipid (e.g., cholesterol), and e) a nucleic acid (e.g., mRNA) under conditions that allow formation of nanoparticles that comprise the nucleic acid.

In embodiments, the targeting agent is added before the nanoparticles are formed, e.g., wherein the targeting agent is added at the same time as the one or more of (e.g., all of) cationic lipid, phospholipid, lipid-anchored PEG, structural lipid, and nucleic acid are combined. In embodiments, the targeting agent is added after the nanoparticles are formed.

In embodiments, upon uptake, the erythroid cell expresses a gene encoded by the nucleic acid (e.g., mRNA). In embodiments, the nucleic acid is not retained after one or more subsequent rounds of cell division.

In embodiments, the cells are not removed from culture media when they are contacted with the carrier. In embodiments, the cells are in culture media when they are contacted with the carrier. In embodiments, the cells are subjected to agitation, e.g., stirring, when they are contacted with the carrier, or within 1, 2, 5, 10, or 30 minutes after being contacted with the carrier.

In embodiments, the method further comprises formulating the erythroid cells into a pharmaceutical composition suitable for administration to a subject in need thereof.

In embodiments, the method comprises contacting the cells with 1-10, 1-5, 1-3, 1.5-2.5, 1.7-2.1, or about 1.9 pg mRNA per cell.

In embodiments, the method does not comprise one or more of (e.g., all of) electroporation, viral transduction, or hypotonic loading.

In embodiments, uptake of the payload by the cells is greater, e.g., at least 10%, 20%, 30%, 40%, 50%, 60%, 70%, 80%, 90%, 2-fold, 5-fold, or 10-fold greater, than uptake of an otherwise similar payload by otherwise similar cells in the absence of the carrier, e.g., nanoparticle. In embodiments, uptake of the payload by the cells is greater, e.g., at least 10%, 20%, 30%, 40%, 50%, 60%, 70%, 80%, 90%, 2-fold, 5-fold, or 10-fold greater, than uptake of an otherwise similar payload by otherwise similar cells using an otherwise similar carrier, e.g., nanoparticle, that lacks the targeting agent.
In some embodiments, the erythroid cell or population of cells has one or more of the following characteristics:
a) an osmotic fragility of less than 50% cell lysis atO.3%, 0.35%, 0.4%, 045%, or 0.5% NaCl;
b) a cell volume of about 10-200 fL or a cell diameter of between about 1 micron and about 20 microns, between about 2 microns and about 20 microns, between about 3 microns and about 20 microns, between about 4 microns and about 20 microns, between about 5 microns and about 20 microns, between about 6 microns and about 20 microns, between about 5 microns and about 15 microns, or between about 10 microns and about 30 microns;
c) greater than 1%, 2%, 3%, 4%, 5%, 6%, 7%, 8%, 9% or 10% fetal hemoglobin; or at least about20, 25, or 30 pg/cell of hemoglobin; or
d) phosphatidylserine content of the outer leaflet is less than 30%, 25%, 20%, 15%, 10%, or 5% as measured by Annexin V staining.

The disclosure contemplates all combinations of any one or more of the foregoing aspects and/or embodiments, as well as combinations with any one or more of the embodiments set forth in the detailed description and examples.

Although methods and materials similar or equivalent to those described herein can be used in the practice or testing of the present invention, suitable methods and materials are described below. All publications, patent applications, patents, and other references (e.g., sequence database reference numbers) mentioned herein are incorporated by reference in their entirety. For example, all GenBank, Unigene, and Entrez sequences referred to herein, e.g., in any Table herein, are incorporated by reference. Unless otherwise specified, the sequence accession numbers specified herein, including in any Table herein, refer to the database entries current as of August 22, 2017. When one gene or protein references a plurality of sequence accession numbers, all of the sequence variants are encompassed.

### BRIEF DESCRIPTION OF THE DRA-WINGS

Fig. 1A and 1B are graphs showing LDL-R presence at the indicated days of erythroid cell differentiation and maturation, as measured by the percent of cells positive for LDL-R (Fig. lA) or the mean fluorescent intensity (Fig. 1B).
Fig. 2 is a graph showing transferrin uptake at the indicated days of erythroid cell maturation.
Fig. 3 is a diagram of an LNP conjugated to a targeting agent comprising transferrin.

### DETAILED DESCRIPTION OF THE INVENTION

### Definitions

As used herein, the term "antibody molecule" refers to a protein, e.g., an immunoglobulin chain or fragment thereof, comprising at least one immunoglobulin variable domain sequence. The term "antibody molecule" encompasses antibodies and antibody fragments. In an embodiment, an antibody molecule is a multispecific antibody molecule, e.g., a bispecific antibody molecule. Examples of antibody molecules include, but are not limited to, Fab, Fab', F(ab')2, Fv fragments, scFv antibody fragments, disulfide-linked Fvs (sdFv), a Fd fragment consisting of the VH and CH1 domains, linear antibodies, single domain antibodies such as sdAb (either VL or VH), camelid VHH domains, multi-specific antibodies formed from antibody fragments such as a bivalent fragment comprising two Fab fragments linked by a disulfide bridge at the hinge region, an isolated epitope binding fragment of an antibody, maxibodies, minibodies, nanobodies, intrabodies, diabodies, triabodies, tetrabodies, v-NAR and bis-scFv.

A "cell surface receptor," as used herein, refers to a protein on the surface of a cell which can bind to a binding partner (e.g., an endogenous ligand or a synthetic binding partner such as an antibody). In embodiments, the cell surface receptor is a transmembrane protein. In embodiments, the cell surface receptor is capable of signal transduction.

As used herein, a "combination therapy" or "administered in combination" means that two (or more) different agents or treatments are administered to a subject as part of a treatment regimen for a particular disease or condition. The treatment regimen includes the doses and periodicity of administration of each agent such that the effects of the separate agents on the subject overlap. In some embodiments, the delivery of the two or more agents is simultaneous or concurrent and the agents may be co-formulated. In other embodiments, the two or more agents are not co-formulated and are administered in a sequential manner as part of a prescribed regimen. In some embodiments, administration of two or more agents or treatments in combination is such that the reduction in a symptom, or other parameter related to the disorder is greater than what would be observed with one agent or treatment delivered alone or in the absence of the other. The effect of the two treatments can be partially additive, wholly additive, or greater than additive (e.g., synergistic). Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination may be administered by intravenous injection while a second therapeutic agent of the combination may be administered orally.

As used herein, the term "differentiation phase" refers to erythroid cells that are in a stage of differentiation characteristic of erythroid cells in the second step described in paragraph [0721] of International Application WO2015/073587.

As used herein, the term "maturation phase" refers to erythroid cells that are in a stage of maturation characteristic of erythroid cells in the third step described in paragraph [0722] of International Application WO2015/073587.

As used herein, "enucleated" refers to a cell, *e.g*., a reticulocyte or mature red blood cell that lacks a nucleus. In an embodiment an enucleated cell is a cell that has lost its nucleus through differentiation from a precursor cell, e.g., a hematopoietic stem cell (*e.g.*, a CD34+ cell), a common myeloid progenitor (CMP), a megakaryocyte erythrocyte progenitor cell (MEP), a burst-forming unit erythrocyte (BFU-E), a colony-forming unit erythrocyte (CFU-E), a pro-erythroblast, an early basophilic erythroblast, a late basophilic erythroblast, a polychromatic erythroblast, or an orthochromatic erythroblast, or an induced pluripotent cell, into a reticulocyte or mature red blood cell. In an embodiment an enucleated cell is a cell that has lost its nucleus through in vitro differentiation from a precursor cell, *e.g*., a hematopoietic stem cell (*e.g*., a CD34+ cell), a common myeloid progenitor (CMP), a megakaryocyte erythrocyte progenitor cell (MEP), a burst-forming unit erythrocyte (BFU-E), a colony-forming unit erythrocyte (CFU-E), a pro-erythroblast, an early basophilic erythroblast, a late basophilic erythroblast, a polychromatic erythroblast, or an orthochromatic erythroblast, or an induced pluripotent cell into a reticulocyte or mature red blood cell.

"Erythroid cell" as used herein, includes a nucleated red blood cell, a red blood cell precursor, an enucleated mature red blood cell, and a reticulocyte. For example, any of a cord blood stem cell, a CD34+ cell, a hematopoietic stem cell (HSC), a spleen colony forming (CFU-S) cell, a common myeloid progenitor (CMP) cell, a blastocyte colony-forming cell, a burst forming unit-erythroid (BFU-E), a megakaryocyte-erythroid progenitor (MEP) cell, an erythroid colony-forming unit (CFU-E), a reticulocyte, an eryfhrocyte, an induced pluripotent stem cell (iPSC), a mesenchymal stem cell (MSC), a polychromatic normoblast, an orthochromatic normoblast, is an erythroid cell. A preparation of erythroid cells can include any of these cells or a combination thereof. In some embodiments, the erythroid cells are immortal or immortalized cells. For example, immortalized erythroblast cells can be generated by retroviral transduction of CD34+ hematopoietic progenitor cells to express Oct4, Sox2, Klf4, cMyc, and suppress TP53 (*e.g.,* as described in Huang et al., Mol Ther 2013, epub ahead of print September 3). In addition, the cells may be intended for autologous use or provide a source for allogeneic transfusion. In some embodiments, erythroid cells are cultured. In an embodiment an erythroid cell is an enucleated red blood cell.

As used herein, the term "exogenous polypeptide" refers to a polypeptide that is not produced by a wild-type cell of that type or is present at a lower level in a wild-type cell than in a cell containing the exogenous polypeptide. In some embodiments, an exogenous polypeptide is a polypeptide encoded by a nucleic acid that was introduced into the cell, which nucleic acid is optionally not retained by the cell. In some embodiments, an exogenous polypeptide is a polypeptide conjugated to the surface of the cell by chemical or enzymatic means.

As used herein, the term "functionalized cell" refers to a cell comprising an exogenous molecule, wherein the cell comprising the exogenous molecule is capable of delivering a therapeutic effect when administered to a subject

As used herein, the term "helper lipid" refers to a lipid that, when comprised by an LNP, improves stability of the LNP, improves efficiency of the LNP's delivery of a payload (e.g., RNA), or both. In some embodiments, improving stability comprises increasing LNP colloidal stability in vitro, e.g., as described in Cheng X, Lee RJ (2016). The role of helper lipids, in lipid -nanoparticles (LNPs) designed for oligonucleotide delivery. Adv Drug Deliv Rev. 2016 Apr 1;99(Pt A):129-137. In some embodiments, a helper lipid is a structural lipid. In some embodiments, the helper lipid is cholesterol.

As used herein, the term "internalization signal" refers to an amino acid sequence that, in at least one protein, promotes movement of the protein from the cell surface to the interior of the cell, e.g., to a vesicle. An internalization signal may have one or more additional functions.

As used herein, the term "targeting agent" refers to any agent that binds to a target, such as a target cell or a target protein (e.g., a target cell surface receptor of an erythroid cell as described herein). Exemplary targeting agents include, e.g., a polypeptide, a nucleic acid, a carbohydrate, a lipid, a receptor ligand, an antibody, and any combination thereof.

The term "nanoparticle" is used herein to refer to a material structure whose size in at least any one dimension (e.g. x, y, and z Cartesian dimensions) is less than about 1 micrometer (micron), e.g. less than about 500 nm or less than about 200 nm or less than about 100 nm, and greater than about 5 nm. In some embodiments, a nanoparticle has a size in all of x, y, and z Cartesian dimensions that is less than about 1 micron and greater than about 5 nm. In some embodiments, a nanoparticle has a size in all of x, y, and z Cartesian dimensions that is less than about 500 nm or less than about 200 nm or less than about 100 nm, and greater than about 5 nm. In embodiments the size is less than about 70 nm but greater than about 20 nm. A nanoparticle can have a variety of geometrical shapes, e.g. spherical, ellipsoidal, etc. The term "nanoparticles" is used as the plural of the term "nanoparticle."

As used herein, the term "variant" of a polypeptide refers to a polypeptide having at least one sequence difference compared to that polypeptide, e.g., one or more substitutions, insertions, or deletions. In some embodiments, the variant has at least 70%, 80%, 85%, 90%, 95%, 96%, 97%, 98%, or 99% identity to that polypeptide. A variant includes a fragment. In some embodiments, a fragment lacks up to 1, 2, 3, 4, 5, 10, 20. or 100 amino acids on the N-terminus, C-terminus, or both (each independently), compared to the full-length polypeptide.

### Carriers, e.g., lipid nanoparticles

In embodiments, the carrier, e.g., nanoparticle, is a lipid nanoparticle (LNP), solid lipid nanoparticle (SLN), nanostructured lipid carrier (NLC), lipoplex nanoparticle, polyplex nanoparticle, polymer nanoparticle, or hybrid lipid/polymer nanoparticle, as described herein. LNPs, in some embodiments, comprise a neutral lipid, cholesterol, and an ionizable or cationic lipid, and can further comprise a payload, such as a nucleic acid. An exemplary LNP comprises a cationic lipid (e.g., a cationic trialkyl lipid), a neutral lipid (e.g., a phospholipid and/or a PEG-lipid conjugate), and a payload comprising a nucleic acid, e.g., DNA or RNA, e.g., an mRNA, e.g., encapsulated within the cationic and non-cationic lipid. In some embodiments, the LNP further comprises a helper lipid or a structural lipid (e.g., cholesterol).

In some embodiments, the helper lipid is a pH-sensitive anionic helper lipid, such as fatty acids and cholesteryl hemisuccinate (CHEMS). In some embodiments, a helper lipid promotes fusion with the cell membrane of a target cell, e.g., as described in Li, W., & Szoka, F. C., Jr. (2007). Lipid-based nanoparticles for nucleic acid delivery. Pharmaceutical Research, 24, 438-449).

In embodiments, the carrier, e.g., nanoparticle (e.g., LNP), comprises a cationic lipid in combination with a non-cationic lipid. While not wishing to be bound by theory, in some embodiments, the cationic lipid can facilitate endosomal escape for the carrier (e.g., LNP), thereby allowing the carrier to deliver its cargo into an erythroid cell. In some embodiments, the cationic lipid comprises a polar head group (e.g., with a pKa between about 5 and about 8), a linker, and a hydrophobic tail. In some embodiments, the polar head group comprises a primary amine, secondary amine, tertiary amine, quaternary ammonium salt, phosphorous group, guanidino group, arsenic group, imidazole group, or pyridinium group. In some embodiments, the linker comprises an ester, ether, carbamate, or amide linkage. In some embodiments, the hydrophobic tail comprises a saturated or unsaturated aliphatic chain connected to the hydrophilic head by the linker.

In some embodiments, the carrier, e.g., nanoparticle (e.g., LNP) comprises a positively charged cationic lipid or an ionizable cationic lipid. In some embodiments, the cationic lipid is configured with a cationic charge at physiological pH with pKa above 8. In some embodiments, the positively charged cationic lipid comprises a tertiary amine with a pKa of less than 7, e.g., allowing for encapsulation of nucleic acids to be performed at acidic pH with the resulting carrier exhibiting a net neutral charge at physiological pH. In some embodiments, the positively charged cationic lipid is selected from the group of N-[1-(2,3-&,oleyloxy) propyl]-N,N,N-trimethylammonium chloride (DOTMA), dioctadecyl dimethylammonium chloride (DODAC), and 6-lauroxyhexyl omithinate (LHON). In an embodiment, the positively charged cationic lipid comprises DOTMA. In an embodiment, the positively charged cationic lipid comprises DODAC. In an embodiment, the positively charged cationic lipid comprises LHON.

In some embodiments, the cationic lipid comprises one or more of stearylamine, 1,3-dimyristoylamidopropane-2-[bis(2-dimethylaminoethane)] carbamate (e.g., 3β[N-(N', N'-dimethylaminoethane)-carbamoyl] cholesterol (DC-Chol)), 1,2-dioleoyl-3-(dimethylamino)propane (DODAP), 1,2-dioleoyl-3-trimethylammonium-propane (DOTAP), 1,2-dipalmitoyl-3-trimethylammoniumpropane (DPTAP), dioctadecyldimelhyl ammonium bromide (DDAB), cetyltrimethylammonium bromide (CTAB),27 N-[1-(2,3-dioleyloxy) propyl]-N, N, N-tri- methylammonium chloride (DOTMA), 1,2-dioleyloxy-3-dimethylaminopropane (DODMA), 1,2-dilinoleyloxy-3-dimethylaminopropane (DLin-DMA), 1,2-dilinoleyloxy-keto-NN-dimethyl-3-aminopropane (DLin-KDMA), 2,2-dilinoleyl-4-(2-dimethylaminoethyl)-[1,3]-dioxolane (DLin-KC2-DMA, e.g., with a pKa of 6.7), 1,2-dilinoleyloxy-N,N-dimethyl-3-aminopropane (DLin-MC3-DMA, e.g., with a pKa of 6.4), N,N-bis(2-hydroxyethyl)-N-methyl-N-(2-cholesteryloxycarbonyl aminoethyl) ammonium bromide (BHEM-Chol), 1,2-dipalmitoyl-sn-glycero-3-phosphoethanolamine-N-(7-nitro-2,-1,3-benzoxadiazol-4-yl) (NBD-DPPE), Genzyme lipid 67 (GL), 1,2-distearyloxy-N,N-dimethylaminopropane (DSDMA), N,N-distearyl-N,N-dimethylammonium bromide (DDAB), N-(1-(2,3-dioleoyloxy)propyl)-N,N,N-trimethylammonium chloride (DOTAP), 3-(N-(N',N'(-dimethylaminoethane)-carbamoyl)cholesterol (DC-Chol), N-(1,2-dimyristyloxyprop-3-yl)-N,N-dimethyl-N-hydroxyelhyl ammonium bromide (DMRIE), 2,3-dioleyloxy-N-[2(spermine-carboxamido)ethyl]-N,N-dimethyl-1-propanaminiumtrifluoroacetate (DOSPA), dioctadecylamidoglycyl spermine (DOGS), 3-dimethylamino-2-(cholest-5-en-3-beta-oxybutan-4-oxy)-1-(cis,cis-9,12-octadecadienoxy)propane (CLinDMA), 2-[5'-(cholest-5-en-3-beta-oxy)-3'-oxapentoxy)-3-dimethy-1-(cis,cis-9',1-2'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-dilinoleylcarbamyl-3-dimethylaminopropane (DLincarbDAP), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1-línoleoyl-2-linoleyloxy-3-dimethylaminopropane (DLin-2-DMAP), 1,2-dilinoleyloxy-3-trimethylaminopropane chloride salt (DLm-TMA-Cl), 1.2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanedio (DOAP), 1,2-dilinoleyloxo-3-(2, N,N-dimethylamino)ethoxypropane (DLin-EG-DMA), 1,2-dioeylcarbamoyloxy-3-dimethylaminopropane (DO-C-DAP), 1,2-dimyristoleoyl-3-dimethylaminopropane (DMDAP), 1,2-dioleoyl-3-trimethylaminopropane chloride (DOTAP.Cl), dilinoleylmethyl-3-dimethylaminopropionate (DLin-M-C2-DMA; also known as DLin-M-K-DMA or DLin-M-DMA), and mixtures thereof.

In an embodiment, the cationic lipid comprises DC-Chol. In an embodiment, the cationic lipid comprises DODAP. In an embodiment, the cationic lipid comprises DOTAP. In an embodiment, the cationic lipid comprises DPTAP. In an embodiment, the cationic lipid comprises DDAB. In an embodiment, the cationic lipid comprises CTAB. In an embodiment, the cationic lipid comprises DOTMA. In an embodiment, the cationic lipid comprises DODMA. In an embodiment, the cationic lipid comprises DLin-DMA. In an embodiment, the cationic lipid comprises DLin-KDMA. In an embodiment, the cationic lipid comprises DLin-KC2-DMA. In an embodiment, the cationic lipid comprises DLin-MC3-DMA (e.g., with a pKa of 6.4). In an embodiment, the cationic lipid comprises DOGS. In an embodiment, the cationic lipid comprises BHEM-Chol. In an embodiment, the cationic lipid comprises NBD-DPPE. In an embodiment, the cationic lipid comprises Genzyme lipid 67 (GL). In some embodiments, the cationic lipid comprises a lipidoid, e.g., a polyamine core lipidoid, e.g., containing a tertiary amine polar head group (e.g., 98N12-5(1), C12-200, cKK-E12, 503O₁₃, or a combination thereof).

In some embodiments, the carrier, e.g., nanoparticle (e.g., LNP) comprises about 50 mol % to about 90 mol %, about 50 mol % to about 85 mol %, about 50 mol % to about 80 mol %, about 50 mol % to about 75 mol %, about 50 mol % to about 70 mol %, about 50 mol % to about 65 mol %, about 50 mol % to about 60 mol %, about 55 mol % to about 65 mol %, or about 55 mol % to about 70 mol % cationic lipid. In some embodiments, the cationic lipid comprises about 50 mol %, 51 mol %, 52 mol %, 53 mol %, 54 mol %, 55 mol %, 56 mol %, 57 mol %, 58 mol %, 59 mol %, 60 mol %, 61 mol %, 62 mol %, 63 mol %, 64 mol %, or 65 mol % cationic lipid. In other embodiments, the carrier, e.g., nanoparticle (e.g., LNP) comprises about 2 mol % to about 60 mol %, about 5 mol % to about 50 mol %, about 10 mol % to about 50 mol %, about 20 mol % to about 50 mol %, about 20 mol % to about 40 mol %, about 30 mol % to about 40 mol %, or about 40 mol % cationic lipid.

In some embodiments, the carrier, e.g., nanoparticle (e.g., LNP), further comprises a non-cationic lipid or neutral lipid. While not wishing to be bound by theory, in some embodiments, the neutral lipid is a helper lipid, such as a phospholipid, that provides structure to the carrier. Exemplary non-cationic lipid can include, e.g., a phospholipid, a cholesterol, or a mixture of a phospholipid and cholesterol. Exemplary non-cationic lipids can include a lecithin (e.g. soy lecithin), e.g., comprising one or more glycerophospholipids (e.g., phosphatidylcholine (PC), phosphatidylethanolamine (PE), phosphatidylinositol, and phosphatidic acid)), such as a mixture of glycerophospholipids. Representative examples of phospholipids include, but are not limited to, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine, dipalmitoylphosphatidylcholine, dioleoylphosphatidylcholine, distearoylphosphatidylcholine, and dilinoleoylphosphatidylcholine. In some embodiments, the non-cationic lipid can include stearic acid.

In some embodiments, the non-cationic lipid comprises a phosphatidylcholine (PC), such as egg PC. In some embodiments, the PC is selected from 1,2-dipalmitoyl-sn-glycero-3-phosphocholine/cholesterol (DPPC), distearoylphosphatidylcholine (DSPC), 1,2-dilauroyl-sn-glycero-3-phosphocholine (DLPC), EPC, 1,2-Dioleoyl-sn-glycero-3-phosphocholine (DOPC), 1-palmitoyl-2-oleoyl-sn-glycero-3-phosphocholine (POPC), 1,2-Dierucoyl-sn-glycero-3-phosphocholine (DEPC), and a mixture thereof. In some embodiments, the PC comprises 1,2-Dipalmi-toylsn-glycero-3-phosphoethanolamine ((DPPE), e.g., NBD-DPPE, or a mixture of a PC lipid and DPPE, such as a mixture of one or more PC lipids and DPPE, e.g., NBD-DPPE.

In some embodiments, the non-cationic lipid comprises a phosphatidylethanolamine (PE). In some embodiments, the PE is selected from dioleoyl-phosphatidylethanolamine (DOPE), 1,2-distearoyl-sn-glycero-3-phosphoethanolamine (DSPE), 1,2-dipalmitoyl-sn-glycero-3-phosphoethanolamine (DPPE)), dimyristoylphosphatidylethanolamine (DMPE), and a mixture thereof.

In some embodiments, the carrier, e.g., nanoparticle (e.g., LNP) comprises about 10 mol % to about 60 mol %, about 20 mol % to about 55 mol %, about 20 mol % to about 45 mol %, about 20 mol % to about 40 mol %, about 25 mol % to about 50 mol 96. about 25 mol % to about 45 mol %, about 30 mol % to about 50 mol %, about 30 mol % to about 45 mol %, about 30 mol % to about 40 mol %, about 35 mol % to about 45 mol %, about 37 mol % to about 42 mol % non-cationic lipid. In some embodiments, the carrier, e.g., nanoparticle (e.g., LNP) comprises about 35 mol %, 36 mol %, 37 mol %, 38 mol %, 39 mol %, 40 mol %, 41 mol %, 42 mol %, 43 mol %, 44 mol %, or 45 mol % non-cationic lipid.

In some embodiments, the carrier, e.g., nanoparticle (e.g., LNP),comprises a lipid-conjugated hydrophilic polymer. In an embodiment, the lipid-conjugated hydrophilic polymer comprises polyethylene glycol (PEG, e.g., PEG₂₀₀₀ or PEGsooo). In some embodiments, the carrier, e.g., LNP, comprises a lipid-PEG molecule, e.g., a DSPE-PEG molecule. In some embodiments, the DSPE-PEG molecule comprises DSPE-PEG 5000, e.g., DSPE-PEG 5000-Azide. In some embodiments, the DSPE-PEG molecule comprises PEG 2000, e.g., PEG 2000-Azide, e.g., 16:0 PEG 2000-Azide. In some embodiments, the azide moiety is used to covalently bond the PEG to a moiety, e.g., a targeting agent, e.g., a CD71-binding agent While not wishing to be bound by theory, in some embodiments, the PEG lipid conjugate improves bioavailability of the carrier (e.g., by increasing the circulation half-life of the carrier), reduces the immunogenicity of the carrier, and/or promotes formation of small, stable carriers (e.g., a LNP). In an embodiment, a PEG lipid conjugate can have a short acyl chain (e.g., C₁₄). In another embodiment, a PEG lipid conjugate can have a long acyl chain (e.g., C₁₈). Exemplary lipid-conjugated hydrophilic-polymers can include, but are not limited to, PEG, PLA [poly(lactide)], PAcM [poly(N-acryloylmorpholine )], PVP [poly(vinylpyrrolidone )], PLA [poly(lactide )], PG [poly(glycolide)], POZO [poly(2-methyl-2-oxazoline)], PVA [poly(vinyl alcohol)], HPMC (hydroxypropylmethylcellulose ), PEO [poly( ethylene oxide)), chitosan [poly(D-glucosamine)], PAA [poly(aminoacid)], polyHEMA (Poly(2 hydroxyethylmethacrylate)], and co-polymers thereof.

In some embodiments, the carrier, e.g., nanoparticle (e.g. a LNP), comprises a PEG-lipid conjugate selected from a PEG-diacylglycerol (PEG-DAG) conjugate, a PEG-dialkyloxypropyl (PEG-DAA) conjugate, a PEG-phospholipid conjugate, a PEG-ceramide (PEG-Cer) conjugate, and a mixture thereof. In some embodiments, the PEG-lipid conjugate inhibits aggregation of particles including the carrier, In some embodiments, the PEG lipid conjugate comprises a PEG-DSPE conjugate, e.g., a methoxyPEG-1,2-distearoyl-sn-glycero-3- phosphoethanolamine (mPEG-DSPE), a maleimide-terminated PEG-1,2-distearoyl-sn-glycero-3-phosphoethanolamine (Mal-PEG-DSPE) conjugate, a DSPE-PEG₂₀₀₀-COOH conjugate, a DSPE-PEG₂₀₀₀ conjugate, or a DSPE-PEG₂₀₀₀-biotin conjugate. In some embodiments, the PEG-DAA conjugate comprises a PEG-dimyristyloxypropyl (PEG-DMA) conjugate, a PEG-distearyloxypropyl (PEG-DSA) conjugate, PEG-didecyloxypropyl conjugate, a PEG-dilauryloxypropyl conjugate, a PEG-dimyristyloxypropyl conjugate, a PEG-dipalmityloxypropyl conjugate, a PEG-distearyloxypropyl conjugate, 1-[8'-(1,2-dimyristoyl-3-propanoxy)-carboxamido-3',6'-dioxaoctanyl]carbamoyl-ω-methyl-poly(ethylene glycol) (2KPEG-DMG), and a combination thereof. In some embodiments, the carrier can include a polyethylene glycol conjugated octadecyl-quarternized lysine modified chitosan (PEG-OQLCS), monomethoxypolyethylene glycol (MePEG-OH), monomethoxypolyethylene glycol-succinate (MePEG-S), monomethoxypolyethylene glycol-succinimidyl succinate (MePEG-S-NHS), monomethoxypolyethylene glycol-amine (MePEG-NH2), monomethoxypolyethylene glycol-tresylate (MePEG-TRES), monomethoxypolyethylene glycol-imidazolyl-carbonyl (MePEG-IM), and such compounds containing a terminal hydroxyl group instead of a terminal methoxy group (e.g., HO-PEG-S, HO-PEG-S-NHS, or HO-PEG-NH₂).

In some embodiments, the PEG moiety of the PEG-lipid conjugate of a carrier, e.g., a nanoparticle (e.g., a LNP), may comprise an average molecular weight ranging about 550 daltons to about 10,000 daltons. In some embodiments, the PEG moiety has an average molecular weight of about 750 daltons to about 5,000 daltons (e.g., about 1,000 daltons to about 5,000 daltons, about 1,500 daltons to about 3,000 daltons, about 750 daltons to about 3,000 daltons, or about 750 daltons to about 2,000 daltons). In certain embodiments, the PEG moiety has an average molecular weight of about 2,000 daltons or about 750 daltons.

In some embodiments, the carrier, e.g., nanoparticle (e.g. a LNP), comprises about 0.1 mol % to about 2 mol %, about 0.5 mol % to about 2 mol %, about 1 mol % to about 2 mol %, about 0.6 mol % to about 1.9 mol %, about 0.7 mol % to about 1.8 mol %, about 0.8 mol % to about 1.7 mol %, about 0.9 mol % to about 1.6 mol %, about 0.9 mol % to about 1.8 mol %, about 1 mol % to about 1.8 mol %, about 1 mol % to about 1.7 mol %, about 1.2 mol % to about 1.8 mol %, about 1.2 mol % to about 1.7 mol %, about 1.3 mol % to about 1.6 mol %, or about 1.4 mol % to about 1.5 mol % lipid conjugate (e.g., PEG-lipid conjugate). In some embodiments, the carrier, e.g., nanoparticle (e.g. a LNP), comprises about 0 mol % to about 20 mol %, about 0.5 mol % to about 20 mol %, about 2 mol % to about 20 mol %, about 1.5 mol % to about 18 mol %, about 2 mol % to about 15 mol %, about 4 mol % to about 15 mol %, about 2 mol % to about 12 mol %, about 5 mol % to about 12 mol %, or about 2 mol % lipid conjugate (e.g., PEG-lipid conjugate). In some embodiments, the carrier, e,g., nanoparticle (e.g. a LNP), comprises about 4 mol % to about 10 mol %, about 5 mol % to about 10 mol %, about 5 mol % to about 9 mol %, about 5 mol % to about 8 mol %, about 6 mol % to about 9 mol %, or about 6 mol % to about 8 mol % lipid conjugate (e.g., PEG-lipid conjugate). In some embodiments, the carrier, e.g., nanoparticle (e.g. a LNP). about 5 mol %, 6 mol %, 7 mol %, 8 mol %, 9 mol %, or 10 mol % lipid conjugate (e.g., PEG-lipid conjugate).

In some embodiments, the carrier, e.g., nanoparticle (e.g., LNP) comprises a helper lipid or a structural lipid. In some embodiments, the helper lipid or structural lipid comprises a sterol (e.g., cholesterol or a derivative thereof), a α-tocopherol (e.g., D-α-tocopherol PEG 1000 succinate (TPGS)), dicetyl phosphate, a stearylamine, or a derivative thereof. While not wishing to be bound by theory, in some embodiments, the helper lipid or structural lipid (e.g., cholesterol or a derivative thereof) increases the carrier (e.g., LNP) stability and promotes fusion of the earner with the plasma membrane. In an embodiment, the helper lipid or structural lipid comprises a α-tocopherol (e.g., D-α-tocopherol PEG 1000 succinate (TPGS)). In an embodiment, the helper lipid or structural lipid comprises dicetyl phosphate. In an embodiment, the helper lipid or structural lipid comprises stearylamine.

In some embodiments, the carrier, e.g., nanoparticle (e.g., LNP) comprises about 25 mol to about 25 mol % to about 40 mol %, about 30 mol % to about 45 mol %, about 30 mol % to about 40 mol %, about 27 mol % to about 37 mol %, about 25 mol % to about 30 mol %, or about 35 mol % to about 40 mol % of a helper lipid or a structural lipid (e.g., cholesterol or a derivative thereof). In some embodiments, the carrier, e.g., nanoparticle (e.g., LNP) comprises about 25 mol % to about 35 mol %, about 27 mol % to about 35 mol %, about 29 mol % to about 35 mol %, about 30 mol % to about 35 mol %, about 30 mol % to about 34 mol %, about 31 mol % to about 33 mol %, or about 30 mol %, 31 mol %, 32 mol %, 33 mol %, 34 mol %, or 35 mol % helper lipid or structural lipid (e.g., cholesterol or a derivative thereof).

In embodiments, the nanoparticle is a polymer nanoparticle or hybrid lipid/polymer nanoparticle, as described herein. In some embodiments, the carrier comprises a polymer selected from D,L-lactic-*co*-glycolic acid (PLGA), polylactide (PLA), γ-polyglutamic acid (γ-PGA), polystyrene, maltodextrin, Pluronic 85, a natural polymer (e.g., chitosan), or a combination thereof. In an embodiment, the carrier comprises PLGA. In an embodiment, the carrier comprises γ-PGA. In an embodiment, the carrier comprises polystyrene. In an embodiment, the carrier comprises maltodextrin. In an embodiment, the carrier comprises Pluronic 85. In an embodiment, the carrier comprises a natural polymer, e.g., chitosan. In some embodiments, the carrier comprises PLGA and/or Pluronic 85 as a polymer core with a lipid shell comprising a lipid layer (e.g., stearic acid) and a lipid-PEG-ligand (e.g., Tf-PEG-oleic acid (Tf-PEG-OA)), as described in Zhu et al., Biomiedicine & Pharmacotherapy 86 (2017) 547-554, which is hereby incorporated by reference in its entirety. In some embodiments, the carrier comprises a γ-PGA-co-PLA-DPPE copolymer, γ-PGA-MAL-PLA-DPPE copolymer, or a γ-PGA-MAL-PLA-DPPE copolymer, e.g., with a targeting moiety comprising Tf Pluronic 85/lipid-polymeric nanoparticles, as described in Zhao et al., Colloids and Surfaces B: Biointerfaces 123 (2014) 787-796, which is hereby incorporated by reference in its entirety.

An-exemplary LNP comprises a cationic trialkyl lipid, a non-cationic lipid (e.g., PEG-lipid conjugate and a phospholipid), and an mRNA molecule that is encapsulated within the lipid particle. In embodiments, the phospholipid comprises dipalmitoylphosphatidylcholine (DPPC), distearoylphosphatidylcholine (DSPC), or a mixture thereof. In embodiments, the PEG-lipid conjugate is selected from the group consisting of a PEG-diacylglycerol (PEG-DAG) conjugate, a PEG-dialkyloxypropyl (PEG-DAA) conjugate, a PEG-phospholipid conjugate, a PEG-ceramide (PEG-Cer) conjugate, and a mixture thereof. In embodiments, the PEG-DAA conjugate is selected from the group consisting of a PEG-didecyloxypropyl (C₁₀) conjugate, a PEG-dilauryloxypropyl (C₁₂) conjugate, a PEG-dimyristyloxypropyl (C₁₄) conjugate, a PEG-dipalmityloxypropyl (C₁₆) conjugate, a PEG-distearyloxypropyl (C₁₈) conjugate, and a mixture thereof. In embodiments, the LNP further comprises cholesterol. Additional LNPs are described, e.g., in US Pat. Pub. 20160256567, which is herein incorporated by reference in its entirety.

Another exemplary LNP can comprise a lipid having a structural Formula (I): 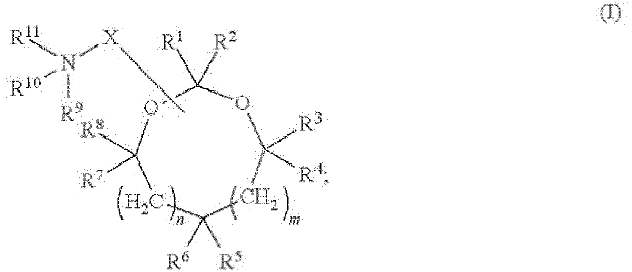 or salts thereof, wherein:
R¹, R², R³, R⁴, R⁵, R⁶, R⁷, and R⁸ are independently selected from the group consisting of hydrogen, optionally substituted C₇-C₃₀ alkyl, optionally substituted C₇-C₃₀ alkenyl and optionally substituted C₇-C₃₀ alkynyl;
provided that (a) at least two of R¹, R², R³, R⁴, R⁵, R⁶, R⁷, and R⁸ are not hydrogen, and (b) two of the at least two of R¹, R², R³, R⁴, R⁵, R⁶, R⁷, and R⁸ that are not hydrogen are present in a 1, 3 arrangement, a 1, 4 arrangement or a 1, 5 arrangement with respect to each other;
X is selected from the group consisting of C₁-C₆ alkyl, C₂-C₆ alkenyl and C₂-C₆ alkynyl;
R⁹, R¹⁰, and R¹¹ are independently selected from the group consisting of hydrogen, optionally substituted C₁-C₇ alkyl, optionally substituted C₂-C₇ alkenyl and optionally substituted C₂-C₇ alkynyl, provided that one of R⁹, R¹⁰, and R¹¹ may be absent; and
n and m are each independently 0 or 1.

For instance, the lipid can comprise one of the following structures: 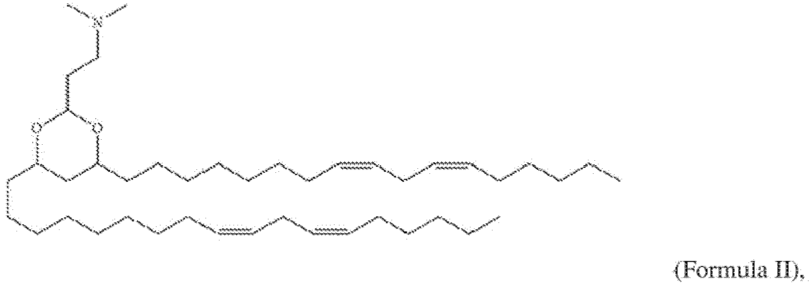 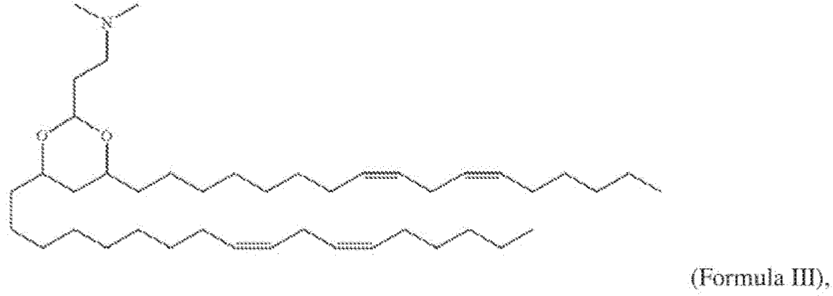 or

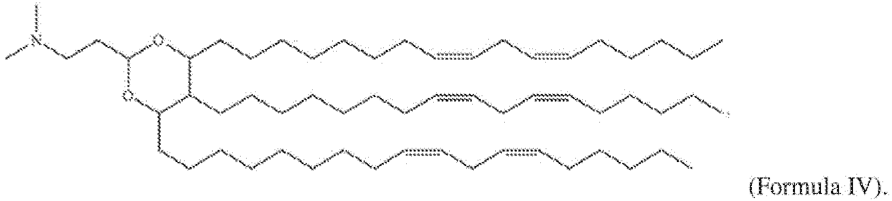

In embodiments, the LNP further comprises a non-cationic lipid such as a phospholipid, cholesterol, or a mixture of a phospholipid and cholesterol. In embodiments, the phospholipid comprises dipalmitoylphosphatidylcholine (DPPC), distearoylphosphatidylcholine (DSPC), or a mixture thereof. Additional LNPs are described, e.g., in US Pat. Pub. 20130064894, which is herein incorporated by reference in its entirety.

Another exemplary LNP comprises: (a) a nucleic acid, e.g., mRNA; (b) a cationic lipid comprising from 50 mol % to 65 mol % (e.g., 52 mol % to 62 mol %) of the total lipid present in the particle; (c) a non-cationic lipid comprising a mixture of a phospholipid and cholesterol or a derivative thereof, wherein the phospholipid comprises from 4 mol % to 10 mol % of the total lipid present in the particle and the cholesterol or derivative thereof comprises from 30 mol % to 40 mol % of the total lipid present in the particle; and (d) a conjugated lipid that inhibits aggregation of particles comprising from 0.5 mol % to 2 mol % of the total lipid present in the particle. In embodiments, the phospholipid comprises dipalmitoylphosphatidylcholine (DPPC), distearoylphosphatidylcholine (DSPC), or a mixture thereof. In embodiments, the conjugated lipid that inhibits aggregation of particles comprises a polyethyleneglycol (PEG)-lipid conjugate. In embodiments, the PEG-lipid conjugate comprises a PEG-diacylglycerol (PEG-DAG) conjugate, a PEG-dialkyloxypropyl (PEG-DAA) conjugate, or a mixture thereof. In embodiments, the PEG-DAA conjugate comprises a PEG-dimyristyloxypropyl (PEG-DMA) conjugate, a PEG-distearyloxypropyl (PEG-DSA) conjugate, or a mixture thereof. Additional LNPs are described, e.g., in US Pat. 8,058,069, which is herein incorporated by reference in its entirety.

Another exemplary LNP comprises a polymer (e.g., polystyrene), a helper lipid or structural lipid (e.g., Choi), a neutral lipid (e.g. EPC), and a PEG moiety (e.g., Mal-PEG-DSPE) conjugated to a targeting agent (e.g., a scFW fragment, e.g., for fibroblast activation protein (FAP)), as described in Messerschmidt J. Control. Release 137 (2009) 69-77, which is herein incorporated by reference in its entirety. Another exemplary LNP comprises a polymer (e.g., PLGA), a helper lipid or structural lipid (e.g., Choi), a neutral lipid (e.g. PC), and a PEG moiety (e.g., DSPE-DSPE), as described in Sengupta Nature 436 (2005) 568-572, which is herein incorporated by reference in its entirety.

Another exemplary LNP comprises a polymer (e.g., PLGA), a helper lipid or structural lipid (e.g., Chol), a neutral lipid (e.g. PC), a PEG moiety (e.g., PEG-OQLCS), and a targeting agent (e.g., FA-OQLCS), as described in Sengupta Nature 436 (2005) 568-572, which is hereby incorporated by reference in its entirety. Additional polymer LNPs including, e.g., FA-OQLCS, are described in Zhao et al. Eur. J. Pharm. Biopharm. 81 (2012) 248-256), which is hereby incorporated by reference in its entirety.

Another exemplary LNP comprises a positively charged cationic lipid (e.g., LHON), a first targeting agent conjugated to PEG-PE (e.g., transferrin-PEG-PE), and a second targeting agent conjugated to PEG-PE (dexamethasone-PEG-PE), as described in Wang et al., International Journal of Nanomedicine 2012:7 2513-2522, which is hereby incorporated by reference in its entirety.

Another exemplary LNP comprises a cationic lipid (e.g., N,N-distearyl-N,N-dimethylammonium bromide (DDAB), a non-cationic lipid (e.g., PC, e.g., soybean PC), and a targeting agent conjugated to PEG-PE (e.g., transferrin-PEG-PE), as described in Han et al., International Journal of Nanomedicine 2014:9 4107-4116, which is hereby incorporated by reference in its entirety.

Another exemplary LNP comprises a polymer (e.g., PLGA), a neutral lipid (e.g. PC), and a targeting agent conjugated to PEG-PE (e.g., transferrin-PEG-PE), as described in Guo et al.. Oncology Letters 9: 1065-1072, 2015, which is hereby incorporated by reference in its entirety.

Another exemplary LNP comprises a cationic lipid (e.g., DOTAP), a neutral lipid (e.g., DOPE), a helper lipid or structural lipid (cholesterol), a PEG moiety (e.g., DSPE-PEG2000-COOH), and a targeting agent (e.g. transferrin), as described in Sharma et al., Pharm. Res. 31 (2014) 1194-1209. In certain embodiments, an LNP comprises DOPE, DOTAP, DSPE-PEG2000-COOH, and cholesterol at a molar ratio of 45:45:4:2.

Another exemplary LNP comprises a neutral lipid (e.g., DSPC), a helper lipid or structural lipid (cholesterol), a PEG moiety (e.g., DSPE-PEG or DSPE-PEG-Maleimide), and a targeting agent (e.g. an Anti-human TfR MAb (e.g., OX26) and/or lactoferrin), as described in De Luca et al. 2015 Int. J. Pharm. 479 (2015) 129-137. In certain embodiments, an LNP comprises about 5.2 µmol DSPC, about 4.5 µmol cholesterol, about 0.3 µmol DSPE-PEG, and 0.18 µmol DSPE-PEG-Maleimide.

In some embodiments, the carrier, e.g., nanoparticle (e.g., LNP), has a mean diameter of about 5 nm to about 500 nm, e.g., 10 nm to about 300 nm, about 20 nm to about 200 nm, about 25 to about 250 nm, about 30 nm to about 150 nm, about 40 nm to about 150 nm, about 50 nm to about 150 nm, about 60 nm to about 130 nm, about 70 nm to about 110 nm, or about 70 to about 90 nm.

In some embodiments, the carrier, e.g., nanoparticle (e.g., LNP), has a lipid to payload ratio (mass/mass ratio) of about 1:1 to about 100:1, about 1:1 to about 50:1, about 2:1 to about 25:1, about 3:1 to about 20:1, about 5:1 to about 15:1, or about 5:1 to about 10:1. In some embodiments, the carrier, e.g., nanoparticle (e.g., LNP), has a lipid to payload ratio (mass/mass ratio) of about 10:1 to about 14:1 or about 9:1 to about 20:1. In some embodiments, the carrier, e.g., nanoparticle (e.g., LNP), has a lipid to payload ratio (mass/mass ratio) of about 5:1, about 6:1, about 7:1, about 8:1, about 9:1, about 10:1, about 11:1, about 12:1, about 13:1, about 14:1, about 15:1, about 16:1, about 17:1, about 18:1, about 19:1, or about 20:1.

In some embodiments, the carrier, e.g., nanoparticle (e.g., LNP), has a Zeta-potential (mV) of about -50 to about 50, e.g., about -40 to about 20, about -35 to about 15, about -30 to about 10, about -20 to about 5, about -15 to about -0, or about -10 to about -5. In certain embodiments, the carrier, e.g., nanoparticle (e.g., LNP), has a Zeta-potential (mV) of about -40, about -35, about -30, about -25, about - 20, about -15, about -10, about -5, or about -2.

In embodiments, a formulation of a carrier (e.g., an LNP) comprises a mixing of any of the aforementioned components in an aqueous phase (e.g., a nucleic acid) and an organic phase. In some embodiments, the lipid components comprise an organic phase that is combined with the aqueous phase (e.g., nucleic acid) to self-assemble the carrier and payload (e.g., a nucleic acid). In an embodiment, the lipid components of the carrier encapsulate the nucleic acid, thereby resulting in a favorable conformation in solution. In some embodiments, the carrier and payload self-assembly can be enhanced by, e.g., the use of microfluidics and/or rapid mixing.

### Targeting agents

In some embodiments, suitable targeting agents include, but are not limited to, a polypeptide, a nucleic acid, a carbohydrate, a receptor ligand, an antibody molecule, e.g., an antibody, and any combination thereof. In some embodiments, the cell surface receptor is an endogenous polypeptide, e.g., an endogenous glycoprotein. In other embodiments, the cell surface receptor is an exogenous polypeptide. In some embodiments, the targeting agent is not covalently linked to PEG, e.g., to PEG-PE. In other embodiments, the targeting agent is covalently linked to PEG, e.g., to PEG-PE.

In embodiments, the carrier (e.g., LNP) comprises a targeting agent that binds a cell surface receptor. In some embodiments, the cell surface receptor is a red blood cell protein selected from the group of transferrin receptor (CD71), glycophorin A (GPA or CD235a), C-Kit receptor, (CD1 17), glucose transporter type 1 (GLUT1)), Band 3,Kell, CD45, CD46, CD47, CD55, CD59, and complement receptor 1 (CR1). In some embodiments, the transmembrane domain is selected from a transmembrane domain of GPA, CD71, Kell, or GLUT1. In some embodiments, the cell surface receptor comprises a type I red blood cell transmembrane protein (e.g., EPO Receptor or GPA), a type II red blood cell transmembrane protein (e.g., CD71 or Kell), or a type III red blood cell transmembrane protein (e.g., GLUT1).

In some embodiments, the cell surface receptor is a protein that comprises an internalization signal, e.g., NPXY, FXNPXY (SEQ ID NO: 5), YXXL, [D/E]XXXL[L/I], YXXQ, GDXY, YXXphi (wherein phi is an amino acid residue with hydrophobic side chains, e.g., V, I, L, F, W, Y or M), or a dileueine-dependent signal-sequence motif such as DKphiTLL (SEQ ID NO: 6), LLHV (SEQ ID NO: 7), HLLPM (SEQ ID NO: 8), and DXXLL. Additional internalization signal are described, e.g., in Pandey "Functional roles of short sequence motifs in the endocytosis of membrane receptors" Front Biosci (Landmark Ed). 2009 Jun 1; 14: 5339-5360, which is incorporated herein by reference in its entirety. In embodiments, the internalization signal is disposed in a cytoplasmic domain of the cell surface receptor. In some embodiments, the internalization motif is a motif that binds a clathrin-associated sorting proteins such as adaptor protein-2 (AP-2), e.g., wherein the internalization motif is [D/E]XXXL[L/I] or YXXQ. In some embodiments, the internalization motif is a motif that binds clathrin.

In some embodiments, a carrier (e.g., an LNP) can be transported into an erythroid cell by either direct fusion with a plasma membrane or by endocytosis (e.g., clathrin-dependent endocytosis, caveolin-dependent endocytosis, or micropinocytosis, see, e.g, Sahay et al. Nature Biotechnology. 2013 July 31(7):653-8, which is hereby incorporated by reference in its entirety). In some embodiments, the cell surface receptor is a protein in the clathrin-mediated endocytic pathway, cayeolae-mediated endocytic pathway, or macropinocytosis endocytic pathway.

In an embodiment, carriers are transported by clathrin-dependent endocytosis into an erythroid cell, e.g., due to the expression of the transferrin receptor (CD71). In some embodiments, the cell surface receptor is CD71. in some embodiments, the targeting agent comprises transferrin or a CD71-binding fragment or analog thereof. In some embodiments, the targeting agent is a CD71-binding fragment of transferrin or analog of transferrin. In some embodiments, the targeting agent comprises an anti-CD71 antibody molecule or a fragment thereof. Exemplary anti-CD71 antibody molecule or a fragment thereof include, e.g., 8D3, RI7217, and OX26 as described in Johnsen and Moos, Journal of Controlled Release 222 (2016) 32-46, which is hereby incorporated by reference in its entirety.

In some embodiments, the targeting agent comprises a CD71-binding polypeptide having a sequence of SEQ ID NO: 11 (human transferrin, Uniprot ID P02787, TRFE_HUMAN), or a sequence least 70%, 85%, 80%, 85%, 90%, 95%, 96%, 97%, 98%, or 99% identity thereto.

MRLAVGALLVCAVLGLCLAVPDKTVRWCAVSEHEATKCQSFRDHMKSVIPSDGPSVACVKKASYLDCIRAIAANEAD
AVTLDAGLVYDAYLAPNNLKPVVAEFYGSKEDPQTFYYAVAVVKKDSGFQMNQLRGKKSCHTGLGRSAGWNIPIGLL
YCDLPEPRKPLEKAVANFFSGSCAPCADGTDFPQLCQLCPGCGCSTLNQYFGYSGAFKCLKDGAGDVAFVKHSTIFE
NLANKADRDQYELLCLDNTRKPVDEYKDCHLAQVPSHTVVARSMGGKEDLIWELLNQAQEHFGKDKSKEFQLFSSPH
GKDLLFKDSAHGFLKVPPRMDAKMYLGYEYVTAIRNLREGTCPEAPTDECKPVKWCALSHHERLKCDEWSVNSVGKI
ECVSAETTEDCIAKIMNGEADAMSLDGGFVYIAGKCGLVPVLAENYNKSDNCEDTPEAGYFAIAVVKKSASDLTWDN
LKGKKSCHTAVGRTAGWNIPMGLLYNKINHCRFDEFFSEGCAPGSKKDSSLCKLCMGSGLNLCEPNNKEGYYGYTGA
FRCLVEKGDVAFVKHQTVPQNTGGKNPDPWAKNLNEKDYELLCLDGTRKPVEEYANCHLARAPNHAVVTRKDKEACV
HKILRQQQHLFGSNVTDCSGNFCLFRSETKDLLFRDDTVCLAKLHDRNTYEKYLGEEYVKAVGNLRKCSTSSLLEAC
TFRRP (SEQ ID NO: 11)

The crystal structure of a complex between transferrin and the transferrin receptor is described in Eckemoth et al., "How the binding of human transferrin primes the transferrin receptor potentiating iron release at endosomal pH" PNAS August 9, 2011. 108 (32) 13089-13094. As described in Eckenroth et al., residues Pro142, Pro145, Asp166, and Phel67 of the N2 subdomain of human transferrin interact with residues of the transferrin receptor. Additional contacts are made by the C1 subdomain of human transferrin, including Asp356 and Cys368. This well characterized binding interface assists one of skill in the art to design transferrin truncations and variants that retain transferrin receptor-binding activity.

In further embodiment, the clathrin-dependent endocytosis in an erythroid cell is dependent on Apolipoprotein E (ApoE). In some embodiments, transfection of an erythroid cell with a carrier (e.g., an LNP) includes the addition of ApoE to the erythroid cell. In some embodiments, the carrier (e.g., an LNP) is included in a bioreactor including an erythroid cell, e.g., the erythroid cell is exposed one or more times (e.g., one, two, three, four, five, six, seven, eight, nine or more times) to the carrier in the bioreactor.

In some embodiments, the targeting agent binds iron. In other embodiments, the targeting agent does not bind iron. In some embodiments, the targeting agent is less than 600, 500, 400, 300, 200, 100, 50, 40, 30, 20, or 10 amino, acids in length. In some embodiments, the targeting agent comprises a CD71-binding amino acid sequence of HAIYPRH (SEQ ID NO: 9) or THRPPMWSPVWP (SEQ ID NO: 10), or a sequence with no more than 1, 2, or 3 alterations relative thereto, e.g., wherein an alteration comprises an insertion, deletion, or substitution or any combination thereof. In some embodiments, the amino acid sequence (e.g., HAIYPRH (SEQ ID NO: 9) or THRPPMWSPVWP (SEQ ID NO: 10)) is covalently linked to PEG, e.g., to PEG-PE. In some embodiments, the targeting agent (e.g., HAIYPRH (SEQ ID NO: 9) or THRPPMWSPVWP (SEQ ID NO: 10)) is not covalently linked to PEG, e.g., to PEG-PE. In some embodiment, the carrier (e.g., LNP) comprises a targeting agent (e.g., HAIYPRH (SEQ ID NO: 9)) conjugated to a PEG chain, poly(l-lysine), and cationic lipid ((e.g., DOTAP) see, e.g., Gao et al. Biochemistry, 1996, 35 (3), pp 1027-1036, which is hereby incorporated by reference in its entirety). In some embodiments, the amino acid sequence (e.g., HAIYPRH (SEQ ID NO: 9) or THRPPMWSPVWP (SEQ ID NO: 10)) binds to the transferrin receptor (TfR), e.g., at a site that is distinct from the binding site of transferrin (see, e.g., Lee et al., Eur. J. Biochem. 268, 2004±2012 (2001), which is hereby incorporated by reference in its entirety).

In some embodiments, the cell surface receptor is Erythropoietin receptor (EPO-R). In some embodiments, the targeting agent comprises an agent that can bind to the EPO-R, e.g., an EPO molecule. An EPO molecule includes, for example, full-length EPO protein or peptide fragments, e.g., functional fragments, thereof. In some embodiments, an EPO molecule includes, e.g., human EPO, EPO from other species, or EPO analogs, such as Epoetin alfa, Epoetin beta, or Darbepoetin alfa. In an embodiment, the EPO molecule is a mammalian EPO molecule, *e.g.,* a human, murine, or rat EPO molecule. In an embodiment, the EPO molecule is a human EPO molecule. In an embodiment, the EPO molecule can be naturally occurring (*e.g*., endogenous EPO purified from cells), or synthetic (*e.g*., recombinant EPO which is exogenously expressed and purified, e.g., from cells).

In an embodiment, the human EPO molecule comprises a polypeptide comprising an amino acid sequence of: 



 (SEQ ID NO: 1),
 or an amino acid sequence having at least 80%, 85%, 90%, 95%, 97%, 98%, or.99% identity to a polypeptide of SEQ ID NO: 1.

In an embodiment, the human EPO molecule comprises a polypeptide comprising an amino acid sequence of: 
APPRLICDSRVLQRYLLEAKEAENITTGCAEHCSLNENITVPDTKVNFYAWKR
MEVGQQAVEVWQGLALLSEAVLRGQALLVNSSQPWEPLQLHVDKAVSGLRSLTTLLR
ALGAQKEAISPPDAASAAPLRTITADTFRKLFRVYSNFLRGKLKLYTGEACRTGDR (SEQ
ID NO: 2),
 or an amino acid sequence having at least 80%, 85%, 90%, 95%, 97%, 98%, or 99% identity to a polypeptide of SEQ ID NO: 2.

In an embodiment, the EPO molecule is a rat EPO molecule comprising a polypeptide comprising an amino acid sequence of: 
MGVPERPTLLLLLSLLLIPLGLPVLCAPPRLICDSRVLERYILEAKEAENVTMGCAEGPR
LSENITVPDTKVNFYAWKRMKVEEQAVEVWQGLSLLSEAILQAQALQANSSQPPESLQL
HIDKAISGLRSLTSLLRVLGAQKELMSPPDATQAAPLRTLTADTFCKLFRVYSNFLRGKL
KLYTGEACRRGDR (SEQ ID NO: 3)
 or an amino acid sequence having at least 80%, 85%, 90%, 95%, 97%, 98%, or 99% identity to a polypeptide of SEQ ID NO: 3.

In an embodiment, the EPO molecule is a mouse EPO molecule comprising a polypeptide comprising an amino acid sequence of: 
MGVPERPTLLLLLSLLLIPLGLPVLCAPPRLICDSRVLERYILEAKEAENVTMGCAEGPR
LSENITVPDTKVNFYAWKRMEVEEQAIEVWQGLSLLSEAILQAQALLANSSQPPETLQL
HIDKAISGLRSLTSLLRVLGAQKELMSPPDTTPPAPLRTLTVDTFCKLFRVYANFLRGKL
KLYTGEVCRRGDR (SEQ ID NO: 4)
 or an amino acid sequence having at least 80%, 85%, 90%, 95%, 97%, 98%, or 99% identity to a polypeptide of SEQ ID NO: 4.

In some embodiments, the nanoparticle further comprises a second targeting agent, e.g., a targeting agent described herein. In some embodiments, the second targeting agent binds the first cell surface receptor or a second cell surface receptor, e.g., wherein the cell surface receptor is a cell surface receptor described herein. In some embodiments, the second targeting agent binds to an EPO-R. In an embodiment, the second targeting agent is an EPO molecule. In some embodiments, the first cell surface receptor comprises transferrin or a CD71-binding fragment or analog thereof and the second targeting agent comprises an EPO molecule.

In some embodiments, the second targeting agent binds to a folate receptor. In an embodiment, the second targeting agent is folate. In some embodiments, the first cell surface receptor comprises transferrin or a CD71-binding fragment or analog thereof and the second targeting agent comprises folate. In some embodiments, the cell surface receptor does not bind to a folate receptor (e.g., the cell surface receptor is not folate).

In some embodiments, the second targeting agent binds to an antigen. In an embodiment, the second targeting agent is an aptamer, e.g., an aptamer that targets a membrane antigen. In some embodiments, the first cell surface receptor comprises transferrin or a CD71-binding fragment or analog thereof and the second targeting agent comprises an aptamer, e.g., an aptamer that targets a membrane antigen. In some embodiments, the cell surface receptor does not bind an antigen (e.g., the cell surface receptor is not an aptamer, e.g., an aptamer that targets a membrane antigen).

In some embodiments, the second targeting agent binds to a αvβ3 integrin receptor, In an embodiment, the second targeting agent is a cyclic peptide, e.g., Arg-Gly-Asp (RGD) peptide-labeled chitosan nanoparticle (RGD-CH-NP) as described in Han et al., Clinical Cancer Research, 16(15), 3910-3922, 2010, which is hereby incorporated by reference in its entirety. In some embodiments, the first cell surface receptor comprises transferrin or a CD71-binding fragment or analog thereof and the second targeting agent comprises a cyclic peptide, e.g., RGD-CH-NP. In some embodiments, the cell surface receptor does not bind the αvβ3 integrin receptor (e.g., the cell surface receptor is not a cyclic peptide, e.g.. RGD-CH-NP).

In some embodiments, the carrier, e.g., lipid nanoparticle, does not contain a targeting agent that binds a glucocorticoid receptor, e.g., does not comprise dexamethasone. In some embodiments, the carrier, e.g., lipid nanoparticle, does not contain a targeting agent that binds a sigma receptor, In some embodiments, the carrier, e.g., lipid nanoparticle, does not contain a targeting agent that binds a fibroblast activation protein (FAP).

### Payload

In some embodiments, the payload comprises a nucleic acid, e.g., DNA or,RNA, e.g., an mRNA. In some embodiments, the RNA comprises one or both of a 5' cap and a 3' poly-A tail.

In some embodiments, the payload comprises a polypeptide. In some embodiments, the polypeptide is a soluble protein, a transmembrane protein, or a lipid-anchored protein.

In some embodiments, the payload comprises a small molecule. In some embodiments, the small molecule is a chemotherapeutic agent, e.g., a mitotic inhibitor or spindle poison (e.g., a taxane, e.g., docetaxel or paclitaxel), e.g., a nucleoside analogue (e.g., gemcitabine or 5-FU); an aromatase inhibitor (e.g., 7α-APTADD); a DNA replication inhibitor, e.g.. a topoisomerase inhibitor (e.g., an anthracycline, e.g., artemisinin, irinotecan, camptothecin, or doxorubicin); an antibiotic (e.g., levofloxacin or fluoroquinolone); a phenol (e.g., a dihydrostilbenoid, e.g., a combretastatin, e.g., combretastatin A-4); an iron oxide nanoparticle (e.g., an Fe₃O₄ nanoparticle or an Fe₂O₃ nanoparticle); a neuronal receptor agonist (e.g., a NK3 receptor agonist; e.g., senktide); or any combination thereof.

In some embodiments, the payload comprises an inorganic molecule. In some embodiments, the payload comprises e.g., an alkylating or alkylating-like agent (e.g., cisplatin). In an embodiment, the payload comprises an isotope (e.g., an isotope of yttrium or indium, e.g., Yttrium-90 or Indium-111).

In some embodiments, the carrier further comprises a second payload. In some embodiments, the payload comprises a nucleic acid, e.g., RNA, and the second payload comprises a second nucleicacid, e.g., a second RNA. In some embodiments, the payload comprises a polypeptide, and the second payload comprises a second polypeptide. In some embodiments, the payload comprises a small molecule, and the second payload comprises a small molecule. In some embodiments, the payload comprises a nucleic acid, e.g., RNA, and the second payload comprises a polypeptide. In some embodiments, the payload comprises a nucleic acid, e.g., RNA, and the second payload comprises a small molecule as described herein. In some embodiments, the payload comprises a polypeptide, and the second payload comprises a small molecule. In some embodiments, the carrier further comprises a third payload, e.g., a payload described herein. In some embodiments, the carrier comprises at least 4, 5, 10, 20, 50, or 100 different payloads, e.g., payloads described herein.

In some embodiments, the payload comprises an enzyme (e.g., asparaginase or PAL) and the second payload comprises a transporter for a substrate or metabolite of the enzyme (e.g., an amino acid transporter).

In embodiments, the carrier (e.g., an LNP) does not include a chemotherapeutic agent, e.g., a mitotic inhibitor or spindle poison (e.g., a taxane, e.g., docetaxel or paclitaxel), e.g., an alkylating or alkylating-like agent (e.g., cisplatin), e.g., a nucleoside analogue (e.g., gemcitabine or 5-FU); an aromatase inhibitor (e.g., 7a-APT ADD); a DNA replication inhibitor, e.g., a topoisomerase inhibitor (e.g., an anthracycline, e.g., artemisinin, irinotecan, camptothecin, or doxorubicin); an antibiotic (e.g., levofloxacin or fluoroquinolone); a phenol (e.g., a dihydrostilbenoid, e.g., a combretastatin, e.g., combretastatin A-4); an isotope (e.g., an isotope of yttrium or indium, e.g., Yttrium-90 or Indium-111); an iron oxide nanoparticle (e.g., an Fe₃O₄ nanoparticle or an Fe₂O₃ nanoparticle); a neuronal receptor agonist (e.g., a NK3 receptor agonist; e.g., senktide); or a combination thereof. In an embodiment, the carrier does not include a mitotic inhibitor or spindle poison, e.g., a taxane. Exemplary taxanes include, e.g., docetaxel or paclitaxel. In an embodiment, the carrier does not include an alkylating or alkylating-like agent. Exemplary alkylating or alkylating-like agents include, e.g., cisplatin. In an embodiment, the carrier does not include a nucleoside analogue. Exemplary nucleoside analogues include, e.g., gemcitabine or 5-FU. In an embodiment, the carrier does not include an aromatase inhibitor. An exemplary aromatase inhibitor includes, e.g., 7α-APTADD. In an embodiment, the carrier does not include a DNA replication inhibitor, e.g., a topoisomerase inhibitor. Exemplary topoisomerase inhibitors include, e.g., an anthracycline (e.g., artemisinin), irinotecan, camptothecin, or doxorubicin. In an embodiment, the carrier does not include an antibiotic. Exemplary antibiotics include, e.g., levofloxacin or fluoroquinolone. In an embodiment, the carrier does not include a phenol, e.g., a dihydrostilbenoid. An exemplary dihydrostilbenoid includes e.g., a combretastatin, e.g., combretastatin A-4. In an embodiment, the carrier does not include an isotope, e.g., an isotope of yttrium or indium. Exemplary isotopes of yttrium include, e.g., Yttrium-90, and exemplary isotopes of indium include, e.g., Indium-111. In an embodiment, the carrier does not include an iron oxide nanoparticle. Exemplary iron oxide nanoparticle include, e.g., an Fe₃O₄ nanoparticle or an Fe₂O₃ nanoparticle. In some embodiments, the carrier does not include a neuronal receptor agonist (e.g., a NK3 receptor agonist. An exemplary NK3 receptor agonist includes; e.g., senktide.

In some embodiments, the carrier (e.g., an LNP) does not include a combination of a DNA replication inhibitor, e.g., a topoisomerase inhibitor (e.g., an anthracycline, e.g., artemisinin, doxorubicin, irinotecan, camptothecin, or doxorubicin) and a phenol (e.g., a dihydrostilbenoid, e.g., a combretastatin, e.g., combretastatin A-4). In an embodiment, the carrier does not include a combination of doxorubicin and combretastatin. In some embodiments, the carrier does not include a combination of a mitotic inhibitor or spindle poison (e.g., a taxane, e.g., docetaxel or paclitaxel) and an isotope (e.g., an isotope of yttrium or indium, e.g., Yttrium-90 or Indium-111). In an embodiment, the carrier does not include a combination of docetaxel and Yittrium-90. In an embodiment, the carrier does not include a combination of docetaxel and Indium-11 1. In some embodiments, the carrier does not include a combination of a topoisomerase inhibitor (e.g., an anthracycline, e.g., artemisinin, doxorubicin, irinotecan, camptothecin, or doxorubicin) and an iron oxide nanoparticle (e.g., an Fe₃O₄ nanoparticle or an Fe₂O₃ nanoparticle). In an embodiment, the carrier does not include a combination of camptothecin and an Fe₃O₄ nanoparticle. In some embodiments, the carrier does not include a combination of a nucleoside analogue (e.g., gemcitabine or 5-FU) and a mitotic inhibitor or spindle poison (e.g., a taxane, e.g., docetaxel or paclitaxel). In an embodiment, the carrier does not include a combination of gemcitabine (e.g. gemcitabine HCl) and paclitaxel. In some embodiments, the carrier does not include a combination of an alkylating or alkylating-like agent (e.g., cisplatin) and a mitotic inhibitor or spindle poison (e.g.,a taxane, e.g., docetaxel or paclitaxel). In an embodiment, the carrier does not include a combination of cisplatin and paclitaxeL In some embodiments, the carrier does not include a combination of two or more topoisomerase inhibitors (e.g., an anthracycline, e.g., artemisinin, doxorubicin, irinotecan, camptothecin, or doxorubicin). In an embodiment, the carrier does not include a combination of doxorubicin and camptothecin;

### Exemplary exogenous polypeptides and uses thereof

In embodiments, the erythroid cell therapeutics described herein comprise one or more (e.g., 2, 3, 4, 5, 6, 10 or more) different exogenous agents, e.g., exogenous polypeptides, lipids, or small molecules or any combination thereof. In some embodiments, an enucleated erythroid cell therapeutic comprises an exogenous fusion polypeptide comprising two or more different proteins described herein. In some embodiments, an enucleated erythroid cell comprises two or more different exogenous polypeptides described herein. In some embodiments, one or more (e.g., all) of the exogenous polypeptides are human polypeptides or fragments or variants thereof.

One or more of the exogenous proteins may have post-translational modifications characteristic of eukaryotic cells, e.g., mammalian cells, e.g., human cells. In some embodiments, one or more (e.g., 2, 3, 4, 5, or more) of the exogenous proteins are glycosylated, phosphorylated, or both. In vitro detection of glycoproteins can be accomplished on SDS -PAGE gels and Western Blots using a modification of Periodic acid-Schiff (PAS) methods. Cellular localization of glycoproteins can be accomplished utilizing lectin fluorescent conjugates known in the art. Phosphorylation may be assessed by Western blot using phospho-specific antibodies.

Post-translation modifications also include conjugation to a hydrophobic group (e.g., myristoylation, palmitoylation, isoprenylation, prenylation, or glypiation), conjugation to a cofactor (e.g., lipoylation, flavin moiety (e.g., FMN or FAD), heme C attachment, phosphopantetheinylation, or retinylidene Schiff base formation), diphthamide formation, ethanolamine phosphoglycerol attachment, hypusine formation, acylation (e.g. O-acylation, N-acylation, or S-acylation), formylation, acetylation, alkylation (e.g., methylation or ethylation), amidation, butyrylation, gamma-carboxylation, malonylation, hydroxylation, iodination, nucleotide addition such as ADP-ribosylation, oxidation, phosphate ester (O-linked) or phosphoramidate (N-linked) formation, (e.g., phosphorylation or adenylylation), propionylation, pyroglutamate formation, S-glutathianylation, S-nitrosylation, succinylation, sulfation, ISGylation, SUMOylation, ubiquitination, Neddylation, or a chemical modification of an amino acid (e.g., citrullination, deamidation, eliminylation, or carbamylation), formation of a disulfide bridge, and racemization (e.g., of proline, serine, alanine, or methionine). In embodiments, glycosylation includes the addition of a glycosyl group to arginine, asparagine, cysteine, hydroxylysine, serine, threonine, tyrosine, or tryptophan, resulting in a glycoprotein. In embodiments, the glycosylation comprises, e.g., O-linked glycosylation or N-linked glycosylation.

In some embodiments, one or more of the exogenous polypeptides is a fusion protein, e.g., is a fusion with an endogenous red blood cell protein or fragment thereof, e.g., a transmembrane protein, e.g., GPA or a transmembrane fragment thereof. In some embodiments, one or more of the exogenous polypeptides is fused with a domain that promotes dimerization or multimerization, e.g., with a second fusion exogenous polypeptide, which optionally comprises a dimerization domain. In some embodiments, the dimerization domain comprises a portion of an antibody molecule, e.g., an Fc domain or CH3 domain. In some embodiments, the first and second dimerization domains comprise knob-in-hole mutations (e.g., a T366Y knob and a Y407T hole) to promote heterodimerization.

### Exemplary cell surface receptors

In an embodiment, the cell surface receptor is expressed on an enucleated erythroid cell. In one embodiment, the cell surface receptor includes CD71 or a fragment thereof, e.g., a fragment of CD71 that can be targeted with a carrier. In one embodiment, the cell surface receptor includes CD335a or a fragment thereof, e.g., a fragment of CD235a that can be targeted with a carrier. In one embodiment, the cell surface receptor includes GLUT1 or a fragment thereof, e.g., a fragment of GLUT1 that can be targeted with a carrier. In one embodiment, the cell surface receptor includes Band 3 or a fragment thereof, e.g., a fragment of Band 3 that can be targeted with a carrier. In one embodiment, the cell surface receptor includes Kell or a fragment thereof, e.g., a fragment of Kell that can be targeted with a carrier. In one embodiment, the cell surface receptor includes CD45 or a fragment thereof, e.g., a fragment of CD45 that can be targeted with a carrier. In one embodiment, the cell surface receptor includes CD46 or a fragment thereof, e.g., a fragment of CD46 that can be targeted with a carrier. In one embodiment, the cell surface receptor includes CD47 or a fragment thereof, e.g., a fragment of CD47 that can be targeted with a carrier. In one embodiment, the cell surface receptor includes CD55 or a fragment thereof, e.g., a fragment of CD55 that can be targeted with a carrier. In one embodiment, the cell surface receptor includes CD59 or a fragment thereof, e.g., a fragment of CD59 that can be targeted with a carrier. In one embodiment, the cell surface receptor includes CR1 or a fragment thereof, e.g., a fragment of CR1 that can be targeted with a carrier. In some embodiments, the carrier includes a targeting agent that binds to a cell surface receptor that undergoes endocytic recycling (e.g. CD71).

### Physical characteristics of enucleated erythroid cells

In some embodiments, the erythroid cells described herein have one or more (e.g., 2, 3, 4, or more) physical characteristics described herein, e.g., osmotic fragility, cell size, hemoglobin concentration, or phosphatidylserine content. While not wishing to be bound by theory , in some embodiments an enucleated erythroid cell that expresses an exogenous protein has physical characteristics that resemble a wild-type, untreated erythroid cell. In contrast, a hypotonically loaded erythroid cell sometimes displays aberrant physical characteristics such as increased osmotic fragility, altered cell size, reduced hemoglobin concentration, or increased phosphatidylserine levels on the outer leaflet of the cell membrane.

In some embodiments, the enucleated erythroid cell comprises an exogenous protein that was encoded by an exogenous nucleic acid that was not retained by the cell, has not been purified, or has not existed fully outside an erythroid cell. In some embodiments, the erythroid cell is in a composition that lacks a stabilizer.

### Osmotic fragility

In some embodiments, the enucleated erythroid cell exhibits substantially the same osmotic membrane fragility as an isolated, uncultured erythroid cell that does not comprise an exogenous polypeptide. In some embodiments, the population of enucleated erythroid cells has an osmotic fragility of less than 50% cell lysis at 0.3%, 0.35%, 0.4%, 0.45%, or 0.5% NaCl. Osmotic fragility can be assayed using the method of Example 59 of WO201 5/073587, which is herein incorporated by reference in its entirety.

### Cell size

In some embodiments, the enucleated erythroid cell has approximately the diameter or volume as a wild-type, untreated erythroid cell.

In some embodiments, the population of erythroid cells has an average diameter of about 4, 5, 6, 7, or 8 microns, and optionally the standard deviation of the population is less than 1, 2, or 3 microns. In some embodiments, the one or more erythroid cell has a diameter of about 4-8, 5-7, or about 6 microns. In some embodiments, the diameter of the erythroid cell is less than about 1 micron, larger than about 20 microns, between about 1 micron and about 20 microns, between about 2 microns and about 20 microns, between about 3 microns and about 20 microns, between about 4 microns and about 20 microns, between about 5 microns and about 20 microns, between about 6 microns and about 20 microns, between about 5 microns and about microns or between about 10 microns and about 30 microns. Cell diameter is measured, in some embodiments, using an Advia 120 hematology system.

In some embodiment the volume of the mean corpuscular volume of the erythroid cells is greater than 10 fL, 20 fL, 30 fL, 40 fL, 50 fL, 60 fL, 70 fL, 80 fL, 90 fL, 100 fL, 110 fL, 120 fL, 150 fL, 140 fL, 150 fL, or greater than 150 fL. In one embodiment the mean corpuscular volume of the erythroid cells is less than 30 fL, 40 fL, 50 fL, 60 fL, 70 fL, 80 fL, 90 fL, 100 fL, 110 fL, 120 fL, 130 fL, 140 fL, 150 fL, 160 fL, 170 fL, 180 fL, 190 fL, 200 fL, or less than 200 fL. In one embodiment the mean corpuscular volume of the erythroid cells is between 80 - 100, 100-200, 200-300, 300-400, or 400-500 femtoliters (fL). In some embodiments, a population of erythroid cells has a mean corpuscular volume set out in this paragraph and the standard deviation of the population is less than 50, 40, 30, 20, 10, 5, or 2 fL. The mean corpuscular volume is measured, in some embodiments, using a hematological analysis instrument, eg., a Coulter counter.

### Hemoglobin concentration

In some embodiments, the enucleated erythroid cell has a hemoglobin content similar to a wild-type, untreated erythroid cell. In some embodiments, the erythroid cells comprise greater than 1%, 2%, 3%, 4%, 5%, 6%, 7%, 8%, 9% or greater than 10% fetal hemoglobin. In some embodiments, the erythroid cells comprise at least about 20, 22, 24, 26, 28, or 30 pg, and optionally up to about 30 pg, of total hemoglobin. Hemoglobin levels are determined, in some embodiments, using the Drabkin's reagent method of Example 33 of WO2015/073587, which is herein incorporated by reference in its entirety.

### Phosphatidylserine content

In some embodiments, the enucleated erythroid cell has approximately the same phosphatidylserine content on the outer leaflet of its cell membrane as a wild-type, untreated erythroid cell. Phosphatidylserine is predominantly on the inner leaflet of the cell membrane of wild-type, untreated erythroid cells, and hypotonic loading can cause the phosphatidylserine to distribute to the outer leaflet where it can trigger an immune response. In some embodiments, the population of erythroid cells comprises less than about 30, 25, 20, 15, 10, 9, 8, 6, 5, 4, 3, 2, or 1% of cells that are positive for Annexin V staining. Phosphatidylserine exposure is assessed, in some embodiments, by staining for Annexin-V-FITC, which binds preferentially to,PS, and measuring FITC fluorescence by flow cytometry, e.g., using the method of Example 54 of WO2015/073587, which is herein incorporated by reference in its entirety.

### Other characteristics

In some embodiments, the population of erythroid cells comprises at least about 50%, 60%, 70%, 80%, 90%, or 95% (and optionally up to 90 or 100%) of cells that are positive for GPA. The presence of GPA is detected, in some embodiments, using FACS.

In some embodiments, the erythroid cells have a half-life of at least 0.5, 1, 2, 7, 14, 30, 45, or 90 days in a subject.

In some embodiments, a population of cells comprising erythroid cells comprises less than about 10, 5, 4,3, 2, or 1% echinocytes.

In some embodiments, an erythroid cell is enucleated, e.g., a population of cells comprising erythroid cells used as a therapeutic preparation described herein is greater than 50%, 60%, 70%, 80%, 90% enucleated. In some embodiments, a cell, e.g., an erythroid cell, contains a nucleus that is non-functional, e.g., has been inactivated.

### Methods of manufacturing enucleated erythroid cells

Methods of manufacturing enucleated erythroid cells comprising (e.g., expressing) an exogenous agent (e.g., polypeptides) are described, e.g., in WO2015/073587 and WO2015/153102, each of which is incorporated by reference in its entirety.

In some embodiments, hematopoietic progenitor cells, e.g., CD34+ hematopoietic progenitor cells, are contacted with a nucleic acid or nucleic acids encoding one or more exogenous polypeptides, and the cells are allowed to expand and differentiate in culture.

In some embodiments, the two or more polypeptides are encoded in a single nucleic acid, e.g. a single vector. In embodiments, the single vector has a separate promoter for each gene, has two proteins that are initially transcribed into a single polypeptide having a protease cleavage site in the middle, so that subsequent proteolytic processing yields two proteins, or any other suitable configuration. In some embodiments, the two or more polypeptides are encoded in two or more nucleic acids, e.g., each vector encodes one of the polypeptides.

The nucleic acid may be, e.g., DNA or RNA.

In some embodiments, a method herein further comprises a step such as transduction, electroporation, sortagging, hypotonic loading, or click chemistry.

A number of viruses may be used as gene transfer vehicles including retroviruses, Moloney murine leukemia virus (MMLV), adenovirus, adeno-associated virus (AAV), herpes simplex virus (HSV), lentiviruses such as human immunodeficiency virus 1 (HIV 1), and spumaviruses such as foamy viruses, for example.

In some embodiments, the cells are produced using sortagging, e.g., as described in WO2014/183071 or WO2014/183066, each of which is incorporated by reference in its entirety.

Erythroid cells described herein can also be produced using coupling reagents to link an agent (e.g., an exogenous polypeptide) to a cell. For instance, click chemistry can be used. Coupling reagents can be used to couple an agent to a cell, for example, when the agent is a complex or difficult to express agent, e.g., a polypeptide, e.g., a multimeric polypeptide; large polypeptide; agent derivatized in vitro, e.g., polypeptide; agent that may have toxicity to, or which are not expressed efficiently in, the erythroid cells.

Thus, in some embodiments, an erythroid cell described herein comprises many as, at least, more than, or about 5,000, 10,000, 50,000, 100,000, 200,000, 300,000, 400,000, 500,000 coupling reagents per cell.. In some embodiments, the erythroid cells are made by a method comprising a) coupling a first coupling reagent to an erythroid cell, thereby making a pharmaceutical preparation, product, or intermediate. In an embodiment, the method further comprises: b) contacting the cell with an agent coupled to a second coupling reagent e.g., under conditions suitable for reaction of the first coupling reagent with the second coupling reagent. In embodiments, two or more agents are coupled to the cell (e.g., using click chemistry). In embodiments, a first agent is coupled to the cell (e.g., using click chemistry) and a second agent comprises a polypeptide expressed from an exogenous nucleic acid.

In some embodiments, the coupling reagent comprises an azide coupling reagent. In some embodiments, the azide, coupling reagent comprises an azidoalkyl moiety, azidoaryl moiety, or an azidoheteroaryl moiety. Exemplary azide coupling reagents include 3-azidopropionic acid sulfo-NHS ester, azidoacetic acid NHS ester, azido-PEG-NHS ester, azidopropylamine, azido-PEG-amine, azido-PEG-maleimide, bis-sulfone-PEG-azide, or a derivative thereof. Coupling reagents may also comprise an alkene moiety, e.g., a transcycloalkene moiety, an oxanorbomadiene moiety, or a tetrazine moiety. Additional coupling reagents can be found in *Click Chemistry Tools* (clickchemistrytools.com/) or Lahann, J (ed) (2009) Click Chemistry for Biotechnology and Materials Science*,* each of which is incorporated herein by reference in its entirety.

In some embodiments, the erythroid cells are expanded at least 1000, 2000, 5000, 10,000, 20,000, 50,000, or 100,000 fold (and optionally up to 100,000, 200,000, or 500,000 fold). Number of cells is measured, in some embodiments, using an automated cell counter.

In some embodiments, the population of erythroid cells comprises at least 30, 35, 40, 45, 50, 55, 60, 65, 70, 75, 80, 85, 90, 95, or 98% (and optionally up to about 80, 90, or 100%) enucleated erythroid cells. In some embodiments, the population of erythroid cells contains less than 1% live enucleated cells, e.g., contains no detectable live enucleated cells. Enucleation is measured, in some embodiments, by FACS using a nuclear stain. In some embodiments, at least 30, 35, 40, 45, 50, 55, 60, 65, 70.75, or 80% (and optionally up to about 70, 80, 90, or 100%) of erythroid cells in the population comprise one or more (e.g., 2, 3, 4 or more) of the exogenous polypeptides. Expression of the polypeptides is measured, in some embodiments, by erythroid cells using labeled antibodies against the polypeptides. In some embodiments, at least 30, 35, 40, 45, 50, 55, 60, 65, 70, 75, or 80% (and optionally up to about 70, 80, 90, or 100%) of erythroid cells in the population are enucleated and comprise one or more (e.g., 2, 3, 4, or more) of the exogenous polypeptides. In some embodiments, the population of erythroid cells comprises about 1×10⁹ - 2×10⁹, 2×10⁹ - 5×10⁹, 5×10⁹ - 1×10¹⁰, 1×10¹⁰ - 2×10¹⁰, 2×10¹⁰ - 5×10¹⁰, 5×10¹⁰ - 1×10¹¹, 1×10¹¹ - 2×10¹¹, 2×10¹¹ - 5×10¹¹, 5×10¹¹ - 1×10¹², 1×10¹² - 2×10¹², 2×10¹² - 5×10¹², or 5×10¹² - 1×10¹³ cells.

### Cells encapsulated in a membrane

In some embodiments, enucleated erythroid cells or other vehicles described herein are encapsulated in a membrane, e.g., semi-permeable membrane. In embodiments, the membrane comprises a polysaccharide, e.g., an anionic polysaccharide alginate. In embodiments, the semipermeable membrane does not allow cells to pass through, but allows passage of small molecules or macromolecules, e.g., metabolites, proteins, or DNA. In embodiments, the membrane is one described in Lienert et al., "Synthetic biology in mammalian cells: next generation research tools and therapeutics" Nature Reviews Molecular Cell Biology 15, 95-107 (2014), incorporated herein by reference in its entirety. While not wishing to be bound by theory, in some embodiments, the membrane shields the cells from the immune system and/or keeps a plurality of cells in proximity, facilitating interaction with each other or each other's products.

### Methods of treatment with compositions herein, e.g., enucleated erythroid cells

Methods of administering enucleated erythroid cells (e.g., reticulocytes) comprising (e.g., expressing) exogenous agent (e.g., polypeptides) are described, e.g., in WO2015/073587 and WO2015/153102, each of which is incorporated by reference in its entirety.

In embodiments, the enucleated erythroid cells described herein are administered to a subject, e.g., a mammal, e.g., a human, Exemplary mammals that can be treated include without limitation, humans, domestic animals (e.g., dogs, cats and the like), farm animals (e.g., cows, sheep, pigs, horses and the like) and laboratory animals (e.g., monkey, rats, mice, rabbits, guinea pigs and the like). The methods described herein are applicable to both human therapy and veterinary applications.

In some embodiments, the erythroid cells are administered to a patient every 1, 2, 3, 4, 5, or 6 months.

In some embodiments, a dose of erythroid cells comprises about 1×10⁹ - 2×10⁹, 2×10⁹ - 5×10⁹, 5×10⁹ - 1×10¹⁰, 1×10¹⁰ - 2×10¹⁰, 2×10¹⁰ - 5×10¹⁰, 5×10¹⁰ - 1×10¹¹, 1×10¹¹ - 2×10¹¹, 2×10¹¹ - 5×10¹¹, 5×10¹¹ - 1×10¹², 1×10¹² - 2×10¹², 2×10¹² - 5×10¹², or 5×10¹² - 1×10¹³ cells.

In some embodiments, the erythroid cells are administered to a patient in a dosing regimen (dose and periodicity of administration) sufficient to maintain function of the administered erythroid cells in the bloodstream of the patient over a period of 2 weeks to a year, e.g., one month to one year or longer, e.g., at least 2 weeks, 4 weeks, 6 weeks, 8 weeks, 3 months, 6 months, a year, 2 years.

In some aspects, the present disclosure provides a method of treating a disease or condition described herein, comprising administering to a subject in need thereof a composition described herein, e.g., an enucleated erythroid cell described herein. In some aspects, the disclosure provides a use of an erythroid cell described herein for treating a disease or condition described herein. In some aspects, the disclosure provides a use of an erythroid cell described herein for manufacture of a medicament for treating a disease or condition described herein.

In embodiments, the disease or condition comprises a proliferative disease such as a cancer, a metabolic disorder (e.g., an enzymatic deficiency such as phenylketonuria or hemophilia), an autoimmune disease, sepsis, or an infectious disease.

### EXAMPLES

### Example 1: CD71-targeted lipid nanoparticle carriers loaded with RNA or DNA

Polyethylene glycol (PEG) is conjugated to a CD71-binding peptide such as HAIYPRH (SEQ ID NO: 9) or THRPPMWSPVWP (SEQ ID NO: 10). The PEG lipid can be; e.g., DSPE-PEG 5000-Azide or 16:0 PEG 2000-Azide. The conjugated PEG is then added to the nanoparticle formulation. 6-lauroxyhexyl ornithinate (LHON), cholesterol, and CD71-binding peptide polyethylene glycol-phosphatidylethanolamine (CD71-binding peptide-PEG) are dissolved in ethanol. The lipid mixture is combined with a 50 mm citrate buffer (pH 4.0) containing DNA or mRNA (e.g., encoding EGFP) at a ratio of 3:1 (aqueous:ethanol) using a microfluidic mixer (Precision Nanosystems, Vancouver, BC). Formulations are concentrated using Amicon Ultra Centrifugal Filters (EMD Millipore, Billerica, MA), pass through a 0.22-mm filter and stored at 4C until use. All formulations are tested for particle size and polydispersity index, e.g., by dynamic light scattering using the Malvern Zetasizer (Worcestershire. UK) using methods described in Chen S et al. Influence of particle size on the in vivo potency of lipid nanoparticle formulations of siRNA. J Control Release. 2016 Aug 10;235:236-44.

Alternatively, the lipid nanoparticles (LNPs) are formulated with a composition of cholesterol, ionizable or cationic lipid, and pegylated lipid with a terminal CLICK handle, such as DBCO or Azide. Nucleic acids such as RNA or DNA are loaded into the previously described LNPs during the formulation process by mixing the two components, lipids and nucleic acids, through a microfluidic mixer (Precision Nanosystems, Vancouver, BC). Once the nucleic acid-LNP is formulated, it is decorated or conjugated with a CD71 targeting peptide (e.g., HAIYPRH (SEQ ID NO: 9), THRPPMWSPVWP (SEQ ID NO: 10)) or whole protein (e.g., transferrin, apo-transferrin, orholo-transferrin) via Azide to DBCO Click chemistry. Post conjugation, the LNPs are concentrated and post-processed through either Amicon Ultra Centrifugal Filters (EMD Millipore, Billerica, MA) or tangential flow filteration (TFF). All formulations are tested for particle size and polydispersity index, e.g., by dynamic light scattering using the Malvern Zetasizer (Worcestershire, UK) using methods described in Chen S et al. Influence of particle size on the in vivo potency of lipid nanoparticle formulations of siRNA. J Control Release. 2016 Aug 10:235:236-44.

CD34+ cells can be expanded and differentiated as described, e.g., in WO2015/073587. Octapeptide/nanoparticle/EGFP mRNA of Example 1 are added to 175,000 erythroid progenitor cells in a 500 ul volume on days 1, 3, 5, 7, and 9 of differentiation and allowed incubate for 24-48 hours at 37 C. Success of the transfection reaction can be quantified by measuring GFP expression by flow cytometry. A population of mock-transfected erythroid cells can serve as a negative control.

### Example 2: Targeted lipid nanoparticle carriers loaded with a protein

lipid nanoparticles are made as described in Example 1, except that nucleic acid is omitted and a protein of interest, e.g., purified EGFP, is included instead.

Erythroid cells are differentiated and contacted with the nanoparticles as described in Example 1. Successful delivery of the eGFP to the erythroid cells can be quantified by measuring EGFP levels by flow cytometry. A population of erythroid cells contacted with nanoparticles lacking EGFP can serve as a negative control.

### Example 3: Targeted lipid nanoparticle carriers loaded with a small molecule

Lipid nanoparticles are made as described in Example 1, except that nucleic acid is omitted and a small molecule of interest, e.g., dexamethasone, is included instead. More specifically, LHON is substituted with dexamethasone-LHON in the reaction with cholesterol and PEG-PE.

Erythroid cells are differentiated and contacted with the nanoparticles as described in Example 1.

Erythroid cells loaded with dexamethasone are tested for cytotoxicity against HepG2 cells. HepG2 cells are seeded in 96-well plates at 8 × 10³ cells/well and incubated for 24 hours, to allow cell attachment. The cells are incubated dexamethasone nanoparticles at various concentrations (10,20, 50, 100, and 200 µg/mL) for 48 hours at 37°C and 5% CO₂ atmosphere, respectively. Cells incubated with nanoparticles lacking dexamethasone are used as a negative control. HepG2 cell viability is assessed using Cell Counting Kit-8 (CCK-8; Dojindo Molecular Technologies, Inc., Gaithersburg, MD), according to the manufacturer's procedures; the absorbance at 450 mm is measured using a microplate reader (Model 680; BIO-RAD, USA). Cells without the addition of CCK-8 are used as a blank to calibrate the spectrophotometer to zero absorbance. The relative cell viability (%) is calculated as: (Abssample - Absblank) / (Abscontrol - Absblank) × 100.

### Example 4: Characteristics of maturing erythroid cells

Cell surface markers were assayed in erythroid cells at different stages of maturation, by flow cytometry. As shown in Table 1, the percentage of GPA-positive cells and Band3-positive cells rose from during maturation, and the percentage of Alpha4 integrin-positive remained high throughout the time course.

**Table 1. Cell surface markers in maturing erythroid cells**

| Stage | Markers | | | |
|---|---|---|---|---|
| | GPA-positive | Alpba4 integrin-positive | Band3-positive | Alpha4 integrin-positive and Band3-positive |
| M0 | 83.9% | 98.0% | 54.6% | 52.9% |
| M3 | 99.0% | 91.4% | 97.8% | 89.6% |
| M5 | 99.5% | 84.2% | 100% | 84.2% |

### Example 5: LDL-R levels decline during erythroid cell maturation

In some cells, the cell surface receptor LDL-R is utilized for passive uptake of lipid nanoparticles (LNPs). In this example, LDL-R levels were assessed in erythroid cells at varying stages of differentiation and maturation. Briefly, wild-type CD34+ cells were differentiated as described herein. Samples of about 0.2 ×10⁶ cells were taken on days D2, D4, D6, and D8 of differentiation, as well as days Ml, M5, and M8 of maturation. Filtered erythroid cells were also tested, to represent fully differentiated cells. The erythroid samples were incubated with 1ul of LDL-R antibody (R&D systems #FAB2148G) in SOul of buffer for 30 minutes. The samples were assayed by FACS (Novocyte). SSC was determined as a measure of cell size, and FITC was quantified as a measure of the amount of LDL-R present on cells. Both the number of LDL-R positive cells and the mean fluorescent intensity (MFI) were measured. A threshold was selected wherein only 1.03% of filtered erythroid cells are positive for LDL-R.

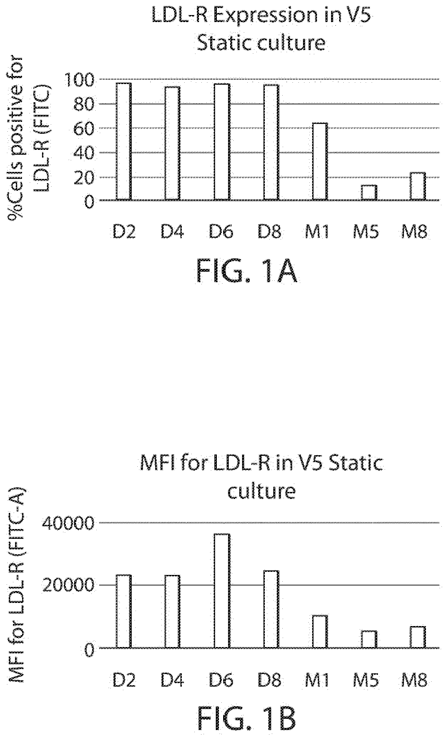

As shown in FIG. 1A, the percentage of erythroid cells that were positive for LDL-R expression remains high through the differentiation phase, but begins to drop upon entry into the maturation phase, until only about 20% of cells show LDL-R expression by day M5. FIG. 1B shows that the mean fluorescent intensity (MFI) of the LDL-R-positive cells also declined during the maturation phase, with a substantial decrease in MFI observed as early as M1. LDL-R expression decreased to its lowest levels (approximately 1%) on fully mature filtered cells.

### Example 6: Transferrin receptor internalization is active throughout erythroid cell maturation

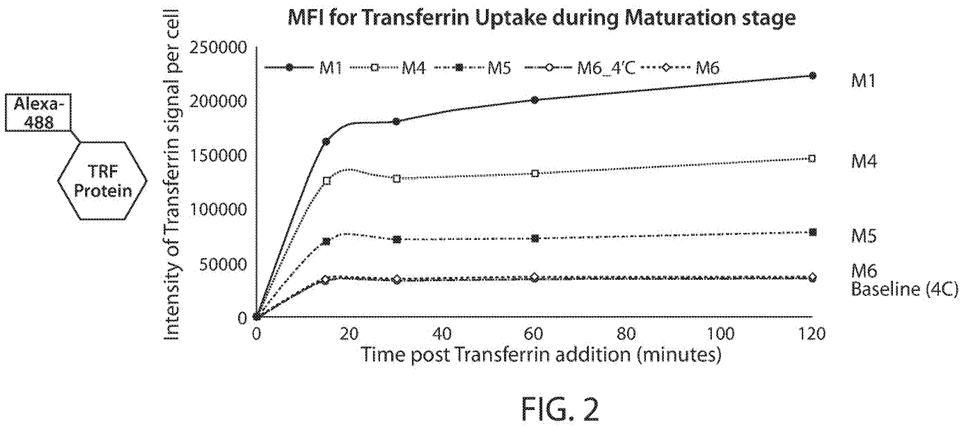

In this example, transferrin receptor (CD71; also referred to herein as TRF) was evaluated for internalization into erythroid cells undergoing maturation. Briefly, wild-type CD34+ cells were differentiated as described herein. Samples of about 0.5×10⁶ cells were taken on days M1, M4, M5, and M6 of maturation. Alexa488-labeled transferrin was obtained from Thermo Fisher Scientific (#T13342). The cells were incubated with the TRF-Alexa488 at a concentration of 1mg/ml in complete media for 0-24 hours at 37 degrees of 4 degrees as indicated in Fig. 2. As shown in FIG. 2, transferrin internalization was active throughout the maturation phase, with the highest activity observed on M1, and activity also being observed at M4 and M5.

### Example 7: A targeting agent comprising a peptide, that binds a cell surface receptor is taken up Into erythroid cells

Uptake of a transferrin-binding peptide was also tested. Wild-type CD34+ cells were differentiated as described herein. At day M2 of maturation/differentiation, FITC-LC-HAIYPRH (a fluorescently labelled peptide that binds the transferrin receptor) was added to a culture of about 0.5 ×10⁶ cells at about 1mg/ml. The labeled peptide was obtained from Anapsec. The cells were cultured in the presence of the labeled peptide for 72 hours at 37 degrees. Internalization of the FITC-LC-HAIYPRH labeled polypeptide by erythroid cells was confirmed by fluorescence microscopy.

### Example 8: Delivery of a payload into erythroid cells using a carrier comprising a targeting agent that binds a cell surface receptor of the erythroid cell

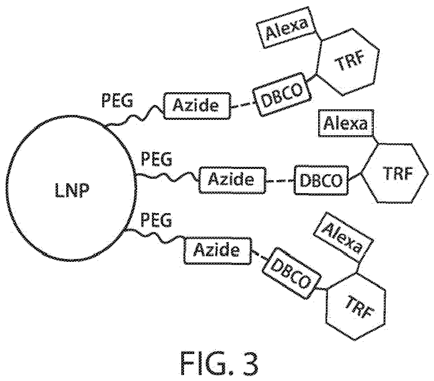

In this example, CD71-targeted lipid nanoparticles (LNPs) were used to introduce a payload (mRNA encoding mCherry), to maturing erythroid cells. LNPs were produced according to the azide conjugation method described in Example 1. Briefly, LNPs were loaded with mRNA encoding mCherry, and conjugated to Alexa488-labeled transferrin (TFR-Alexa488). More specifically, DSPE-PEG 5000-Azide was included in a lipid mixture used to formulate the nanoparticle. The lipid mixture was combined with mCherry-encoding mRNA at a ratio of 3:1 (aqueous:ethanol) inside a microfluidic mixer (Precision Nanosystems Inc., Vancouver, BC). Next, TFR-Alexa488 was labelled with DBCO and then conjugated by the Click chemistry method, as described herein, to the mCherry-loaded LNPs, thereby generating -mCherry-LNP-Azide-DBCO-TFR-Aleza488 (LNP-TFR) (see Fig. 3). Formulations were concentrated using Amicon Ultra Centrifugal Filters (EMD Millipore, Billerica, MA), passed through a 0.22-mm filter, and stored at 4°C until use.

Erythroid cells at stage M2 were starved of transferrin by withdrawing media and incubating in transferrin-free media for two hours. The erythroid cells were then treated with single controls, unconjugated LNPs, or conjugated LNP-TFR. The treated cells were then incubated for 4 days to provide time for internalization and expression of mCherry protein to occur. Detection of Alexa488 indicated delivery of the labeled carrier (CD71 targeted LNP-TFR), and detection of mCherry protein indicated successful delivery and translation of the mRNA. As shown in Table 2, expression of mCherry protein was observed in M6 erythroid cells treated with TRF-conjugated LNPs (LNP-Azide + TFR-Alexa488-DBCO) at markedly higher levels than in negative controls. Indeed, only this treatment group showed greater than 2% of cells being transferrin-high and mCherry-high (28.9%), as shown in Table 2 below.

**Table 2. Percentage of mCherry and transferrin-expressing cells**

| | **TF^{high}** | **TF^{high}** | **TF^{low}** | **TF^{low}** |
|---|---|---|---|---|
| | **mCherry^{low}** | **mCherry^{high}** | **mCherry^{low}** | **mCherry^{high}** |
| **Cells Only** | 0.62 | 0.099 | 98.0 | 1.23 |
| **LNP** | 0.46 | 0.087 | 98.5 | 0.94 |
| **LNP-Azide** | 0.55 | 0.043 | 98.6 | 0.79 |
| **TFR-Alexa488** | 97.7 | 0.81 | 1.44 | 0 |
| **TFR-Alexa488 + DBCO** | 96.8 | 1.45 | 1.74 | 0.039 |
| **LNP + TFR-Alexa488-DBCO** | 99.0 | 0.18 | 0.83 | 0 |
| **LNP-Azide** + **TFR-Alexa488-DBCO** | 71.0 | 28.9 | 0.11 | 0 |

This experiment demonstrates the successful and specific delivery of a payload into erythroid cells using a carrier comprising a targeting agent that binds a cell surface receptor of the erythroid cell. When targeted LNPs were used, over 28 times as many cells expressed the encoded protein compared to non-targeted LNPs (Table 2).

### EMBODIMENTS:

1. An *in vitro* method of introducing a payload into an erythroid cell, comprising:
   contacting an erythroid cell with a carrier, which carrier comprises:
      a) a payload (e.g. a nucleic acid or a polypeptide), and
      b) a targeting agent that binds a cell surface receptor of an erythroid cell,
   under conditions that allow uptake of the payload into the erythroid cell,
   thereby introducing the payload into the erythroid cell.
2. The method of embodiment 1, wherein the carrier comprises a nanoparticle.
3. The method of embodiment 1, wherein the carrier comprises a lipid nanoparticle (LNP).
4. The method of any of the preceding embodiments, wherein the payload comprises a nucleic acid.
5. The method of embodiment 4, wherein the nucleic acid comprises DNA or RNA, e.g., an mRNA.
6. The method of any of the preceding embodiments, wherein the payload comprises a polypeptide.
7. The method of any of the preceding embodiments, wherein the cell surface receptor comprises an internalization signal chosen from NPXY, FXNPXY (SEQ ID NO: 5), YXXL, [D/E]XXXL[L/I], YXXQ, GDXY, and YXXphi.
8. The method of any of the preceding embodiments, wherein the cell surface receptor comprises CD71.
9. The method of embodiment 8, wherein the targeting agent comprises transferrin or a CD71-binding fragment or analog thereof.
10. The method of embodiment 8, wherein the targeting agent has an amino acid sequence of SEQ ID NO: 11 or an amino acid sequence having at least 70%, 85%, 80%, 85%, 90%, 95%, 96%, 97%, 98%, or 99% identity thereto, that binds CD71.
11. The method of any of the preceding embodiments, wherein the targeting agent comprises an amino acid sequence of HAIYPRH (SEQ ID NO: 9).
12. The method of any of the preceding embodiments, wherein the targeting agent is covalently or non-covalently bound to the surface of the carrier.
13. The method of any of the preceding embodiments, wherein the payload comprises a nucleic acid, the carrier comprises a nanoparticle, and wherein the nucleic acid is disposed in the core of the nanoparticle.
14. The method of any of the preceding embodiments, wherein the erythroid cell is in differentiation phase.
15. The method of any of embodiments 1-13, wherein the erythroid cell is in maturation phase.
16. The method of any of embodiments 1-13, wherein the erythroid cell is a pro-erythroblast, basophilic erythroblast, or normoblast (e.g., polychromatic normoblast or orthochromatic normoblast).
17. The method of any of the preceding embodiments, wherein the payload comprises a nucleic acid, and wherein the method further comprises culturing the cell under conditions that allow for translation of the nucleic acid to produce an encoded polypeptide.
18. The method of embodiment 17, wherein at least 10%, 15%, 20%, 25%, or 30% of cells in a population of cells produce the encoded polypeptide.
19. The method of any of the preceding embodiments, which does not comprise contacting the erythroid cells with an agent that competes with the targeting agent for binding to the cell surface receptor (a competing agent).
20. The method of embodiment 19, which comprises withdrawing media containing the competing agent and adding media that does not comprise the competing agent, or adding media that comprises a lower level of the competing agent compared to the withdrawn media.
21. The method of embodiment 19 or 20, wherein the competing agent is transferrin, e.g., iron-bound transferrin.
22. An *in vitro* method of introducing an mRNA into an erythroid cell, comprising:
   contacting an erythroid cell with an LNP, which LNP comprises:
      a) the mRNA, and
      b) a CD71 -binding targeting agent that comprises an amino acid sequence of HAIYPRH (SEQ ID NO: 9) or an amino acid sequence having at least 70%, 85%, 80%, 85%, 90%, 95%, 96%, 97%, 98%, or 99% identity thereto,
   under conditions that allow uptake of the mRNA into the erythroid cell,
   thereby introducing the mRNA into the erythroid cell.
23. A reaction mixture comprising:
   a) a carrier, e.g., a nanoparticle, comprising a payload (e.g., a nucleic acid) and a targeting agent that binds a cell surface receptor of an erythroid cell,
   b) an erythroid cell comprising the cell surface receptor, said cell surface receptor capable of mediating internalization of the carrier.
24. A carrier, e.g., a nanoparticle, comprising:
   a) mRNA, and
   b) a targeting agent that binds a cell surface receptor of an erythroid cell.
25. An erythroid cell comprising a carrier (e.g., nanoparticle) of embodiment 24.
26. The erythroid cell of embodiment 25, wherein the carrier is disposed at the cell surface, or internal to the cell, e.g., in an endocytic vesicle, in an endosome, or in the cytoplasm.
27. An erythroid cell that comprises a payload, made by a process comprising:
   contacting an erythroid cell with a carrier, e.g., a nanoparticle, which carrier comprises:
      a) the payload, and
      b) a targeting agent that binds a cell surface receptor of an erythroid cell,
   under conditions that allow uptake of the payload into the erythroid cell.
28. A preparation comprising at least 10⁵, 10⁶, 10⁷, 10⁸, 10⁹, 10¹⁰, 10¹¹, 10¹², or 10¹³ erythroid cells of any of embodiments 25-27.

## Claims

1. A carrier, e.g., a nanoparticle, comprising:
a) mRNA, and
b) a targeting agent that binds a cell surface receptor of an erythroid cell.

2. The carrier of claim 1, wherein the carrier is a nanoparticle, preferably a lipid nanoparticle (LNP).

3. The carrier of claim 1 or 2, wherein the cell surface receptor is CD71, CD117, GLUT1, Band 3, Kell, CD45, CD46, CD47, CD5, CD59, or complement receptor 1 (CR1), preferably wherein the cell surface receptor is CD71 or CD117, more preferably wherein the cell surface receptor is CD71.

4. The carrier of any one of claims 1-3, wherein the erythroid cell is a cord blood stem cell, a CD34⁺ cell, a hematopoietic stem cell (HSC), a spleen colony forming (CFU-S) cell, a common myeloid progenitor (CMP) cell, a blastocyte colony-forming cell, a burst forming unit-erythroid (BFU-E), a megakaryocyte-erythroid progenitor (MEP) cell, an erythroid colony-forming unit (CFU-E), a reticulocyte, an erythrocyte, an induced pluripotent stem cell (iPSC), a mesenchymal stem cell (MSC), a polychromatic normoblast, or an orthochromatic normoblast.

5. An erythroid cell comprising a carrier of any one of claims 1-4.

6. The erythroid cell of claim 5, wherein the carrier is disposed at the cell surface, or internal to the cell, e.g., in an endocytic vesicle, in an endosome, or in the cytoplasm, and/or
wherein the erythroid cell is a cord blood stem cell, a CD34⁺ cell, a hematopoietic stem cell (HSC), a spleen colony forming (CFU-S) cell, a common myeloid progenitor (CMP) cell, a blastocyte colony-forming cell, a burst forming unit-erythroid (BFU-E), a megakaryocyte-erythroid progenitor (MEP) cell, an erythroid colony-forming unit (CFU-E), a reticulocyte, an erythrocyte, an induced pluripotent stem cell (iPSC), a mesenchymal stem cell (MSC), a polychromatic normoblast, or an orthochromatic normoblast.

7. An erythroid cell that comprises a payload, made by a process comprising:
contacting an erythroid cell with a carrier, e.g., a nanoparticle, which carrier comprises:
a) the payload, and
b) a targeting agent that binds a cell surface receptor of an erythroid cell,
under conditions that allow uptake of the payload into the erythroid cell, optionally wherein the erythroid cell is a cord blood stem cell, a CD34+ cell, a hematopoietic stem cell (HSC), a spleen colony forming (CFU-S) cell, a common myeloid progenitor (CMP) cell, a blastocyte colony-forming cell, a burst forming unit-erythroid (BFU-E), a megakaryocyte-erythroid progenitor (MEP) cell, an erythroid colony-forming unit (CFU-E), a reticulocyte, an erythrocyte, an induced pluripotent stem cell (iPSC), a mesenchymal stem cell (MSC), a polychromatic normoblast, or an orthochromatic normoblast.

8. A preparation comprising at least 10⁵, 10⁶, 10⁷, 10⁸, 10⁹, 10¹⁰, 10¹¹, 10¹², or 10¹³, erythroid cells of any of claims 5-7.

9. A reaction mixture comprising:
a) a carrier, e.g., a nanoparticle, comprising a payload (e.g., a nucleic acid) and a targeting agent that binds a cell surface receptor of an erythroid cell,
b) an erythroid cell comprising the cell surface receptor, said cell surface receptor capable of mediating internalization of the carrier.

10. The reaction mixture of claim 9, wherein the carrier is a nanoparticle, preferably a lipid nanoparticle (LNP), and/or
wherein the payload is a nucleic acid.

11. The reaction mixture of claim 9 or 10, wherein the cell surface receptor is CD71, CD117, GLUT1, Band 3, Kell, CD45, CD46, CD47, CD5, CD59, or complement receptor 1 (CR1), preferably wherein the cell surface receptor is CD71 or CD117, more preferably wherein the cell surface receptor is CD71.

12. The reaction mixture of any one of claims 9-11, wherein the erythroid cell is a cord blood stem cell, a CD34+ cell, a hematopoietic stem cell (HSC), a spleen colony forming (CFU-S) cell, a common myeloid progenitor (CMP) cell, a blastocyte colony-forming cell, a burst forming unit-erythroid (BFU-E), a megakaryocyte-erythroid progenitor (MEP) cell, an erythroid colony-forming unit (CFU-E), a reticulocyte, an erythrocyte, an induced pluripotent stem cell (iPSC), a mesenchymal stem cell (MSC), a polychromatic normoblast, or an orthochromatic normoblast.

13. The erythroid cell of any one of claims 5-7 for use in therapy.

14. The erythroid cell of any one of claims 5-7 for use in a method of treating a proliferative disease such as a cancer, a metabolic disorder (e.g., an enzymatic deficiency such as phenylketonuria or hemophilha), an autoimmune disease, sepsis, or an infectious disease in a subject in need thereof.

15. An *in vitro* method of introducing a payload into an erythroid cell, comprising:
contacting an erythroid cell with a carrier, which carrier comprises:
a) a payload (e.g. a nucleic acid or a polypeptide), and
b) a targeting agent that binds a cell surface receptor of an erythroid cell,
under conditions that allow uptake of the payload into the erythroid cell, thereby introducing the payload into the erythroid cell,
optionally wherein the erythroid cell is a cord blood stem cell, a CD34+ cell, a hematopoietic stem cell (HSC), a spleen colony forming (CFU-S) cell, a common myeloid progenitor (CMP) cell, a blastocyte colony-forming cell, a burst forming unit-erythroid (BFU-E), a megakaryocyte-erythroid progenitor (MEP) cell, an erythroid colony-forming unit (CFU-E), a reticulocyte, an erythrocyte, an induced pluripotent stem cell (iPSC), a mesenchymal stem cell (MSC), a polychromatic The method of claim 13, wherein the carrier comprises a nanoparticle, preferably a lipid nanoparticle (LNP),
optionally wherein the payload comprises a nucleic acid, optionally wherein the nucleic acid comprises DNA or RNA, e.g., an mRNA, optionally wherein the payload comprises a polypeptide.
